# EUROPÄISCHE PATENTSCHRIFT

(11) **EP 0 742 556 B1**
(45) Veröffentlichungstag und Bekanntmachung des Hinweises auf die Patenterteilung: **02.10.2002**
(21) Anmeldenummer: 96810278.0
(22) Anmeldetag: 01.05.1996
(51) Int. Cl.: G11B 7/24, C09B 69/08, D06P 1/00

(54) **Strukturierte Pigmentbeschichtung und deren Herstellung sowie Verwendung**
Structured pigment coating and its manufacture and use
Revêtement pigmentaire structuré et sa fabrication et son utilisation

(30) Priorität: 12.05.1995 CH 139495
(43) Veröffentlichungstag der Anmeldung: 13.11.1996
(73) Patentinhaber: Ciba Specialty Chemicals Holding Inc., 4057 Basel (CH)
(72) Erfinder: Zambounis, John, Dr., 4056 Basel (CH); Hofmann, Manfred, Dr., 1723 Marly (CH)

(56) Entgegenhaltungen:
- EP-A- 0 401 791
- EP-A- 0 459 201
- EP-A- 0 490 817
- EP-A- 0 530 145
- EP-A- 0 615 233
- EP-A- 0 648 770
- EP-A- 0 648 817
- BE-A- 516 435
- BE-A- 557 851
- JOURNAL OF ORGANIC CHEMISTRY, Bd. 22, 1957, Seiten 127-132, XP002012309 C. J PEDERSEN: "Reversible oxidation of phthalocyanines"

## Beschreibung

Die vorliegende Erfindung betrifft ein Verfahren zur Herstellung eines Materials enthaltend ein Substrat und eine unter Verwendung löslicher Pigmentvorläufer erzeugte Pigmentbeschichtung, bestimmte nach dem Verfahren hergestellte Materialien sowie deren Verwendung als Farbfilter oder zur permanenten Speicherung digitaler Information.

Es ist aus EP-648770, EP-648817, Angewandte Chemie 68/4, 133-150 (1956) sowie J. Org. Chem. 22, 127-132 (1957) bekannt, dass organische Pigmente in lösliche, andersfarbige Derivate umgewandelt werden können. Bei diesen Derivaten handelt es sich um Fluoreszenzfarbstoffe, beziehungsweise um Küpenfarbstoffe, welche jedoch für viele färberische Zwecke unerwünschte Eigenschaften aufweisen, beispielsweise schwache Lichtstabilität, hohe Migrationstendenz, tiefer Schmelzpunkt oder Fluoreszenz. Solche in einem Substrat gelöste Farbstoffe können wieder in die Ausgangspigmente zurückgeführt werden, oder in Pigmente anderer Kristallmodifikationen überführt werden.

Hochwertige Pigmente besitzen hingegen hohe Lichtechtheiten und lösen sich nicht oder nur zu einem sehr geringen Teil im zu färbenden Substrat. Organische Pigmente können zwar laut A. B. Marchant, "Optical Recording", 360-371 (Addison-Wesley Publ. Co., 1990), W. Kern & L. Vossen, "Thin Film Processes" (1978) und JP-88/118098 direkt auf Substrate sublimiert (aufgedampft) werden, jedoch erfordert dies wegen der beschränkten Thermostabilität der Pigmente meist Hochvakuum [H. Nakazumi et al., J. Soc. Dyers + Col. 106/11, 367 (1990)].

Die Sublimation ist daher ein sehr langsames und umständliches sowie kostenungünstiges, für die effiziente Massenproduktion pigmentierter Güter ungeeignetes Beschichtungsverfahren. Zudem ist es schwierig, das Kristallwachstum zu steuern, so dass durch Sublimation erhaltene Beschichtungen öfters Pigmentpartikel unerwünschter Korngrösse enthalten, oder nicht im gewünschten Masse homogen und gleichmässig gefärbt sind. Mit der Sublimationsmethode lassen sich keine hochaufgelöste Muster herstellen, und es gibt das Problem an unerwünschten Stellen des Substrats oder der Apparatur niedergeschlagener Pigmentpartikeln.

Pigmente werden deswegen fast immer nur zur Massenfärbung eingesetzt, d.h. sie werden einfach in einem geeigneten Substrat dispergiert, beispielweise in Kunststoffgranulaten, -Folien oder -Fasern, Lackformulierungen oder Drucktinten. Bei der Massenfärbung treten aber Nachteile auf, wie Rheologieprobleme, insbesondere bei sehr feinen Pigmentteilchen, negative Effekte der Pigmente auf die Lichtstabilität des Substrates, oder unbefriedigende Echtheiten der Pigmente im Zusammenhang mit der Natur des Substrates.

Weiter verlangen homogene Färbungen lange Dispersionszeiten des organischen Pigments bei hoher Scherenergie und/oder hoher Temperatur. Dies kann entweder direkt bei der Einverleibung ins Substrat geschehen, oder schon zuvor wie bei der Herstellung von Pigmentpräparaten oder Masterbatches.

Zudem ist es in der Massenfärbung nötig, von einem gegebenen Chromophor je nach Verwendungszweck und beabsichtigtem Resultat auf unterschiedliche Pigmentvarianten zurückzugreifen, wie transparente oder deckende Form, unterschiedliche Oberflächenbehandlungen oder verschiedene Kristallmodifikationen. Das hat eine unnötige und unwirtschaftliche Verbreiterung des an Lager zu haltenden Sortimentes zur Folge.

Bei der Verwendung eines Bindemittels können zudem die darin eingeschlossen Vorläufer mit der Zeit unerwünscht zu farbigen Produkten reagieren.

Das Bedürfnis nach einer einfachen Beschichtungsmethode ohne Verwendung von Bindemitteln konnte also bisher nicht befriedigt werden.

Die vorliegende Erfindung betrifft nun ein Verfahren zur Herstellung eines Materials enthaltend ein Substrat, an dessen Oberfläche mindestens eine aus einem oder mehreren Pigmenten der Formel (I) oder (II) oder Derivaten davon,

**A(D**_{**1**}**)(D**_{**2**}**)**_{**x**} **(I)**

**Pc (II)**

worin A den Rest eines Chromophors der Chinacridon-, Anthrachinon-, Perylen-, Indigo-, Azo-, Chinophthalon-, Isoindolinon-, Isoindolin-, Dioxazin-, Phthalocyanin- oder Diketopyrrolopyrrolreihe bedeutet, der mit D₁ und x D₂ verbundene N-Atome enthält, wobei jedes in A vorhandene N-Atom unabhängig von den anderen mit 0, 1 oder 2 Gruppen D₁ oder D₂ verbunden sein kann,
D₁ und D₂ Wasserstoff, x eine ganze Zahl von 0 bis 4 bedeuten, und
Pc ein Chromophor der Phthalocyanin-Reihe ist,
bestehende Pigmentbeschichtung angebracht ist, durch
(a) Beschichtung des Substrats mit einer Lösung oder Schmelze mindestens eines latenten Pigmentes der Formel (III),

   **A(D**_{**3**}**)(D**_{**4**}**)**_{**x**} **(III)**

   oder eines Derivaten davon,
   oder mindestens eines latenten Pigmentes der Formel (IV), 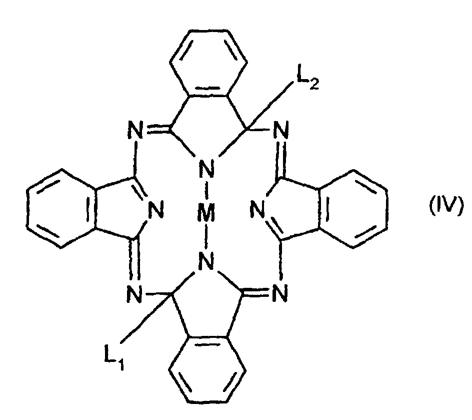 oder eines Stellungsisomeren oder eines Derivaten davon,
   worin in Formel (III) A und x die gleiche Bedeutung haben wie in Formel (I), wobei A mit D₃ und x D₄ verbundene N-Atome enthält und jedes in A vorhandene N-Atom unabhängig von den anderen mit 0, 1 oder 2 Gruppen D₃ oder D₄ verbunden sein kann, und
   D₃ und D₄ unabhängig voneinander Gruppen der Formel 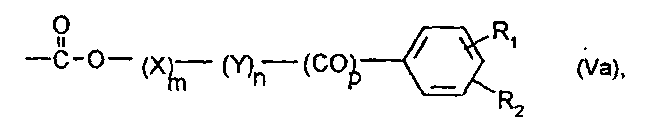 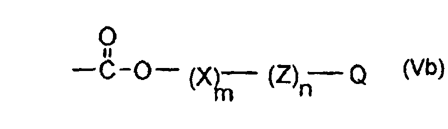 oder 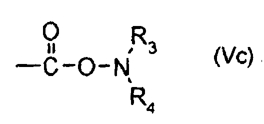 sind,
   und worin in Formel (IV) L₁ und L₂ unabhängig voneinander Halogen, C₁-C₁₈-Alkoxy, C₂-C₁₈-Dialkylamino, C₁-C₁₈-Cycloalkylamino, C₁-C₆-Alkylpiperidino oder Morpholino bedeuten, und M für zwei Wasserstoffe oder ein mindestens zweiwertiges Metallatom steht,
   und
(b) partielle oder vollständige Überführung des latenten Pigmentes in dessen unlösliche Pigmentform durch Abspaltung der Gruppen D₃ und D₄ unter deren Ersatz durch Wasserstoff, oder durch Abspaltung der Gruppen L₁ und L₂, wobei in den Formeln (Va), (Vb) und (Vc)
   - m, n und p: unabhängig voneinander 0 oder 1 sind,
   - X: C₁-C₁₄-Alkylen, C₂-C₁₄-Alkenylen, C₂-C₁₄-Alkinylen, C₄-C₁₂-Cycloalkylen oder C₄-C₁₂-Cycloalkenylen,
   - Y: eine Gruppe -V-(CH₂)_{q}-,
   - Z: eine Gruppe -V-(CH₂)ᵣ-,
   - V: C₃-C₆-Cycloalkylen,
   - q: eine Zahl von 1 bis 6 und
   - r: eine Zahl von 0 bis 6 bedeuten,
   - R₁ und R₂: unabhängig voneinander Wasserstoff, C₁-C₆-Alkyl, C₁-C₄-Alkoxy, Halogen, CN, NO₂, unsubstituiertes oder durch C₁-C₄-Alkyl, C₁-C₄-Alkoxy oder Halogen substituiertes Phenyl oder Phenoxy sind,
   - Q: Wasserstoff, CN, Si(R₁)₃, eine Gruppe C(R₅)(R₆)(R₇),
   worin R₅, R₆ und R₇ unabhängig voneinander Wasserstoff oder
   Halogen sind und mindestens einer der Reste R₅, R₆ und R₇ Halogen bedeutet,
   eine Gruppe 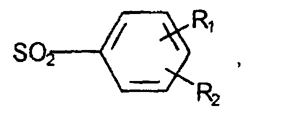 worin R₁ und R₂ die oben angegebene Bedeutung haben,
   eine Gruppe SO₂R₈ oder SR₈, worin R₈ C₁-C₄-Alkyl ist,
   eine Gruppe CH(R₉)₂, worin R₉ unsubstituiertes oder durch C₁-C₄-Alkyl, C₁-C₄-Alkoxy oder Halogen substituiertes Phenyl bedeutet,
   oder eine Gruppe der Formel 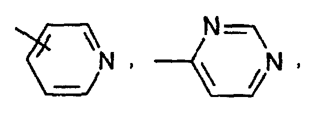 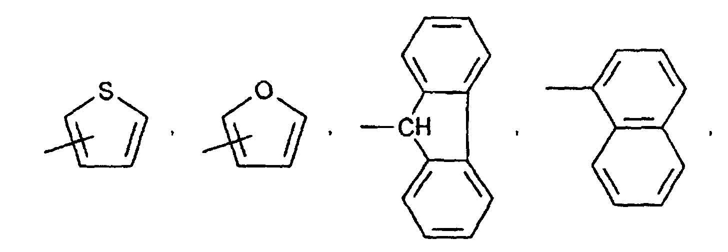 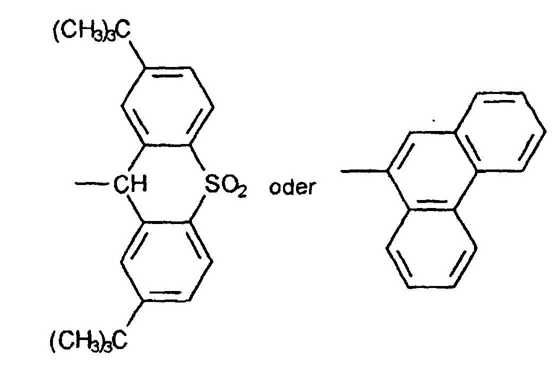 bedeutet, und
   - R₃ und R₄: unabhängig voneinander Wasserstoff, C₁-C₁₈-Alkyl oder eine Gruppe 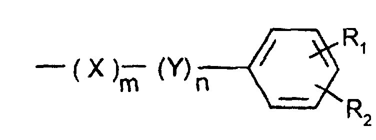 sind,
   worin X, Y, R₁, R₂, m und n die oben angegebene Bedeutung haben, oder R₃ und R₄ zusammen mit dem Stickstoffatom an dem sie gebunden sind einen Pyrrolidinyl-, Piperidinyl- oder
   Morpholinylrest bilden.

Vorzugsweise ist im Farbstoffrest A jedes mit Gruppen D₁, D₂, D₃ oder D₄ verbundenes N-Atom mindestens einer Carbonylgruppe unmittelbar benachbart, oder jedes dieser N-Atome ist mit einer Carbonylgruppe konjugiert. Es ist nicht nötig, und oftmals nicht angezeigt, dass alle N-Atome eines Farbstoffrestes mit Gruppen D₁, D₂, D₃ oder D₄ verbunden sind; vielmehr können A(D₁)(D₂)ₓ und A(D₃)(D₄)ₓ gegebenenfalls weitere 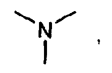 =N-, -NH- oder -NH₂ Gruppen innerhalb des Restes A enthalten.

Umgekehrt können mehr als eine Gruppe D₁, D₂, D₃ oder D₄ mit einem einzigen N-Atom verbunden sein, zum Beispiel zwei, wenn der Chromophor eine Gruppe ―NH₂ enthält, so dass dessen Rest A sowohl ―NH' als auch ―N: sein kann.

A bedeutet den Rest bekannter Chromophore mit der Grundstruktur

**A(H)(H)**_{**x**}**,**

wie beispielsweise 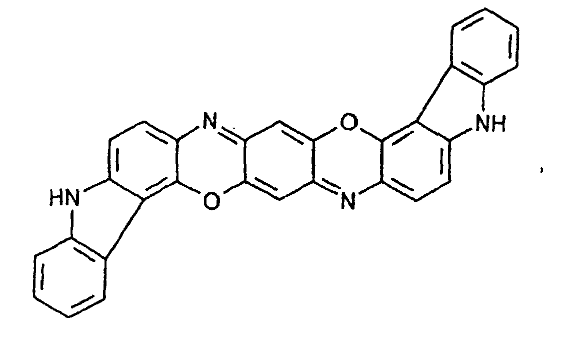 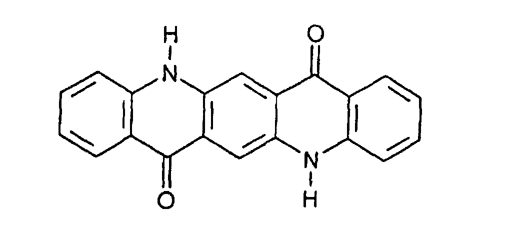 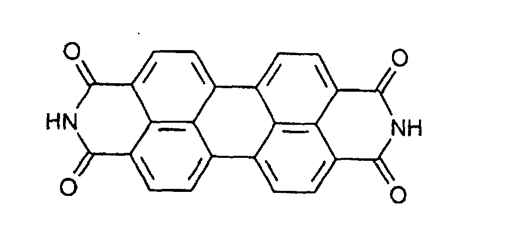 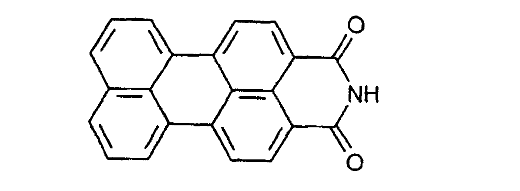 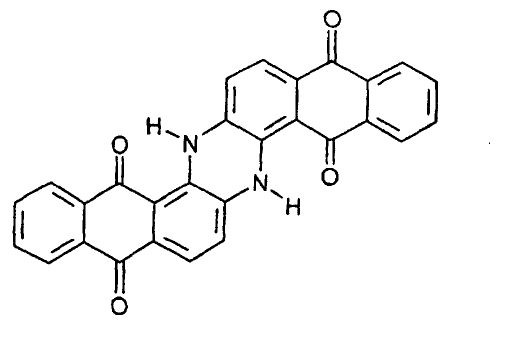 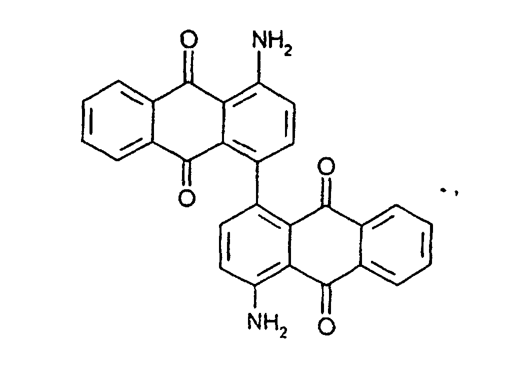 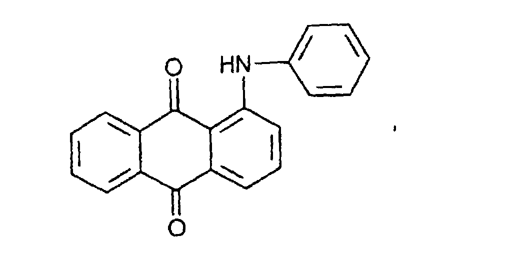 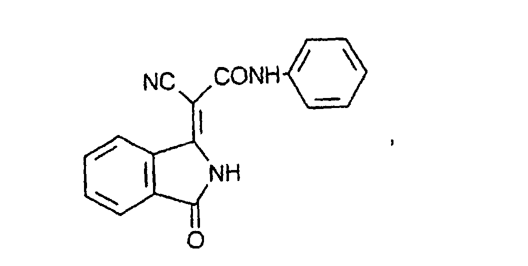 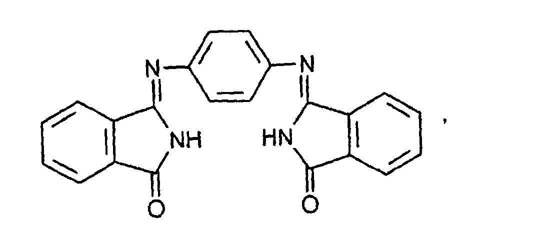 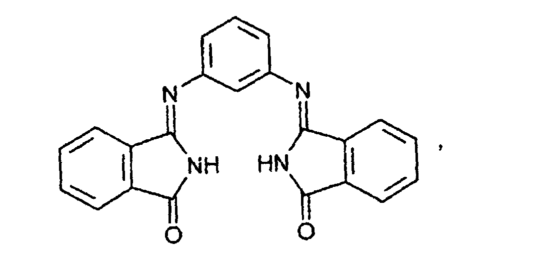 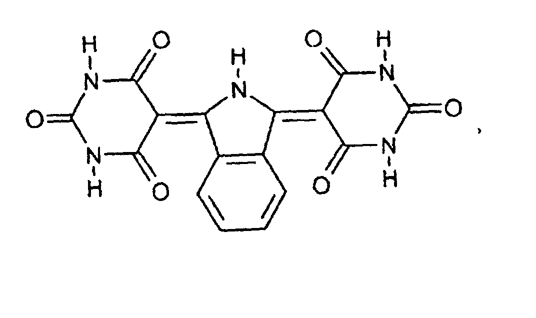 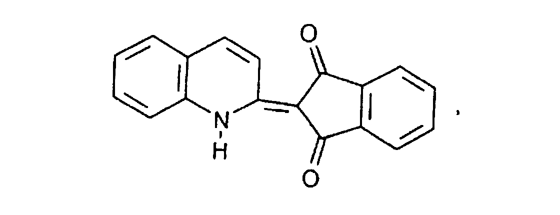 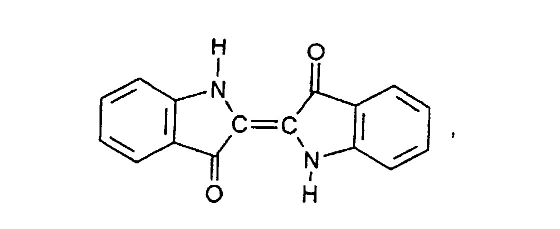 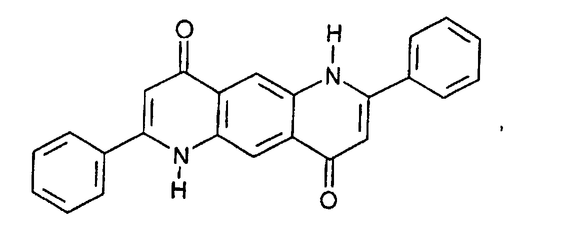 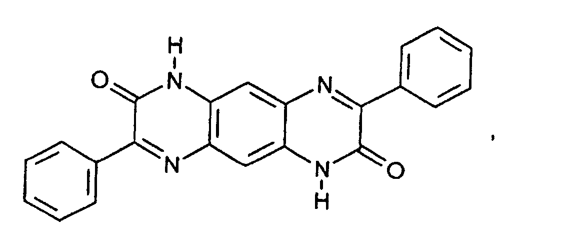 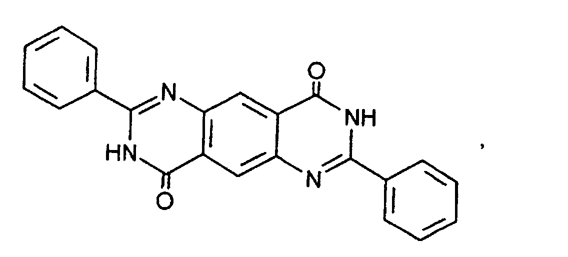 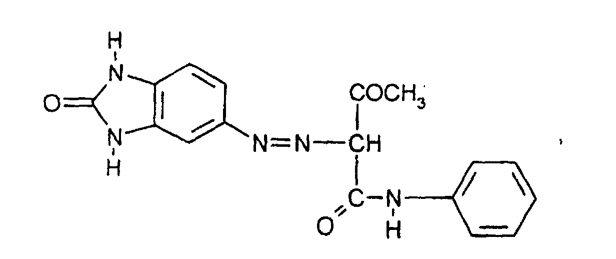 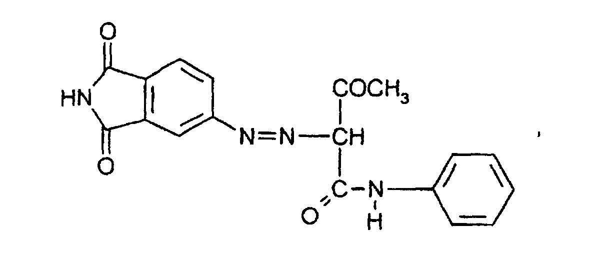 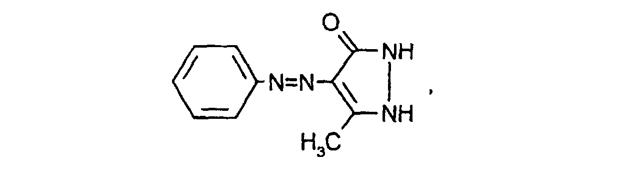 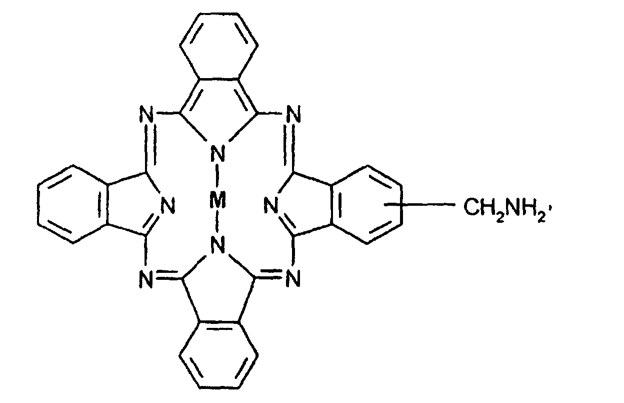 worin M die oben angegebene Bedeutung hat, oder 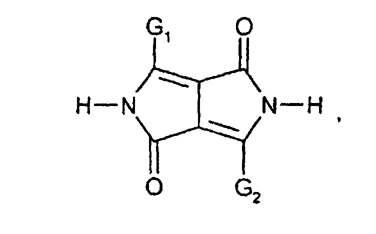 worin G₁ und G₂ unabhängig voneinander für eine Gruppe der Formel 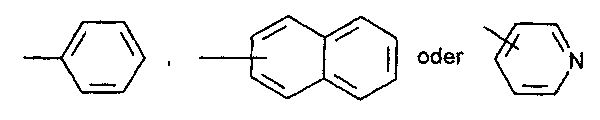 stehen, oder jeweils ein beliebiges davon bekanntes Derivat.

Pc bedeutet ein Phthalocyanin der Grundstruktur 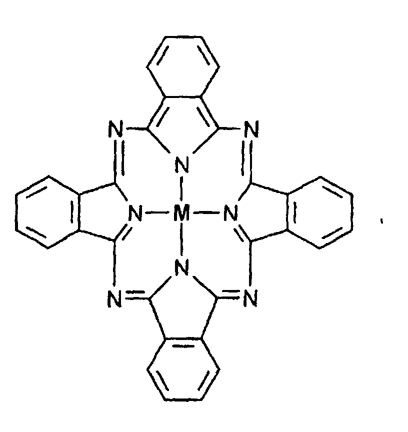 worin M beispielsweise H₂, Mg, Ca, Ti, V, Mn, Fe, Co, Ni, Cu, Zn, Zr, Pd, Cd, Sn, Ce, Hg, Pb oder Bi, insbesondere ein zweiwertiges Metall bedeutet,
oder ein beliebiges davon bekanntes Derivat.

Als Derivate seien beispielsweise einfach oder mehrfach substituierte Chromophore der obengenannten Grundstrukturen benannt, sowie insbesondere bei Phthalocyaninen solche, worin das Metall als Metalloxyd, Metallsalz oder Metallkomplex vorliegt, beispielsweise als -Ti(O)-, -V(O)-, -Fe(OH)- oder -[Co⁺(NH₃)₂]Cl⁻-. Gegebenenfalls kann es sich bei den Substituenten am Kohlenstoffgerüst beispielweise um über eine Direktbindung oder über Carbonyl, Carbonyloxy, Oxycarbonyl, Sulfonyl oder Sulfinyl gebundenes Halogen, Nitro, Amino, Hydroxy, C₁-C₁₈-Alkyl, C₅-C₆-Cycloalkyl, C₁-C₄-Alkoxy, C₁-C₁₈-Alkylthio, C₁-C₁₈-Alkylamino, C₂-C₁₈-Dialkylamino oder C₁-C₁₈-Cycloalkylamino handeln.

Bedeutet X C₁-C₁₄-Alkylen oder C₄-C₁₂-Cycloalkylen, so handelt es sich um ein geradkettiges oder verzweigtes Alkylen oder Cycloalkylen, wie z.B. Methylen, Dimethylen, Trimethylen, 1-Methyl-methylen, 1,1-Dimethyl-methylen, 1-Ethyl-dimethylen, 1,1-Dimethyldimethylen, 1-Ethyl-1-methyl-dimethylen, 1,1-Dimethyl-trimethylen, 2,2-Dimethyl-trimethylen, Tetramethylen, 1,1-Dimethyl-tetramethylen, Hexamethylen, Decamethylen, 1,1-Dimethyldecamethylen, 1,1-Diethyl-decamethylen, Tetradecamethylen, 1,2-Cyclobutylen, 1,2-Cyclopentylen, 1,2-Cyclohexylen, 1,4-Cyclohexylen oder 2,2,6-Trimethyl-1,4-cyclohexylen.

X als C₂-C₁₄-Alkenylen, C₂-C₁₄-Alkinylen oder C₄-C₁₂-Cycloalkenylen bedeutet geradkettiges oder verzweigtes Alkenylen, Alkinylen oder Cycloalkenylen, wie z.B. Vinylen, Allylen, Methallylen, 1-Methyl-2-butenylen, 1,1-Dimethyl-3-butenylen, 2-Butenylen, 2-Hexenylen, 3-Hexenylen, 1-Methyl-2,6-but-3-inylen oder 1,4-Cyclohexylen.

Bedeuten etwaige Substitutenten Halogen, dann handelt es sich z.B. um Jod, Fluor, insbesondere Brom und bevorzugt Chlor.

Bei C₁-C₆-Alkyl handelt es sich beispielsweise um Methyl, Ethyl, n-Propyl, Isopropyl, n-Butyl, sec.-Butyl, tert.-Butyl, n-Amyl, tert.-Amyl, Hexyl und bei C₁-C₁₈-Alkyl zusätzlich z.B. um Heptyl, Octyl, 2-Ethylhexyl, Nonyl, Decyl, Dodecyl, Tetradecyl, Hexadecyl oder Octadecyl.

C₅-C₆-Cycloalkyl bedeutet z.B. Cyclopentyl und insbesondere Cyclohexyl.

C₃-C₆-Cycloalkylen steht z.B. für Cyclopropylen, Cyclobutylen, Cyclopentylen und insbesondere für Cyclohexylen.

C₁-C₄-Alkoxy bedeutet z.B. Methoxy, Ethoxy, n-Propoxy, lsopropoxy, Butyloxy, und C₁-C₁₈-Alkoxy zusätzlich z.B. Hexyloxy, Decyloxy, Dodecyloxy, Hexadecyloxy oder Octadecyloxy.

C₁-C₁₈-Alkylthio steht beispielsweise für Methylthio, Ethylthio, Propylthio, Butylthio, Octylthio, Decylthio, Hexadecylthio oder Octadecylthio.

C₁-C₁₈-Alkylamino bedeutet z.B. Methylamino, Ethylamino, Propylamino, Hexylamino, Decylamino, Hexadecylamino oder Octadecylamino.

C₂-C₁₈-Dialkylamino bedeutet z.B. Dimethylamino, Diethylamino, Methylpropylamino, Ethylhexylamino, Methyldecylamino, Dioctylamino oder Ethylhexadecylamino, wobei die Kohlenstoffatome beider Alkylreste zusammengezählt werden.

C₁-C₁₈-Cycloalkylamino bedeutet z.B. Pyrrolidino, Piperidino, Methylpyrrolidino, Methylpiperidino, Dimethylpyrrolidino, Dimethylpiperidino, Dodecylpyrrolidino, Dodecylpiperidino, bevorzugt Piperidino, 4-Methylpiperidino, 4-Ethylpiperidino, 4-Propylpiperidino und 4-Butylpiperidino.

Von besonderem Interesse ist das oben dargestellte Verfahren, worin latente Pigmente der Formel (III) verwendet werden, worin x 0 oder 1 ist und D₃ sowie D₄ unabhängig voneinander Gruppen der Formel 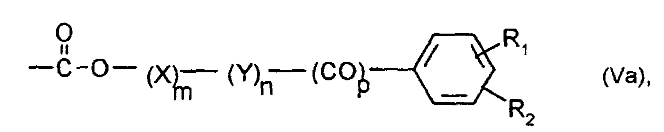 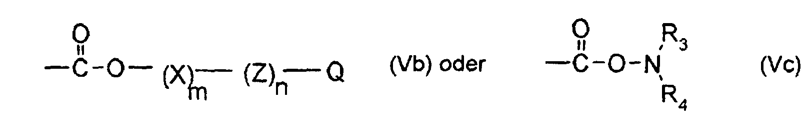 sind,
- n und p: beide gleich 0 sind,
- m: 0 oder 1 bedeutet,
- Q: Wasserstoff, CN, CCl₃, eine Gruppe 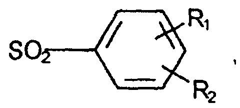 SO₂CH₃ oder SCH₃,
- X: C₁-C₄-Alkylen oder C₂-C₅-Alkenylen ist,
- R₁ und R₂: unabhängig voneinander Wasserstoff, C₁-C₄-Alkyl, Methoxy, Chlor oder NO₂ bedeuten, und
- R₃ und R₄: unabhängig voneinander Wasserstoff, C₁-C₄-Alkyl oder eine Gruppe 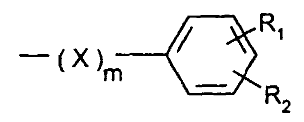 bedeuten, oder R₃ und R₄ zusammen einen Piperidinylrest bilden,
und insbesondere jene, worin x 1 ist und D₃ und D₄ identisch sind und Gruppen der Formel 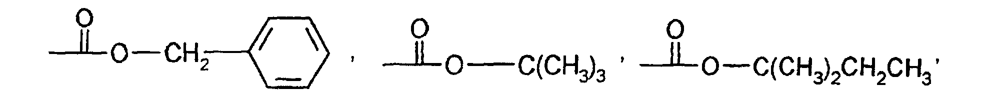 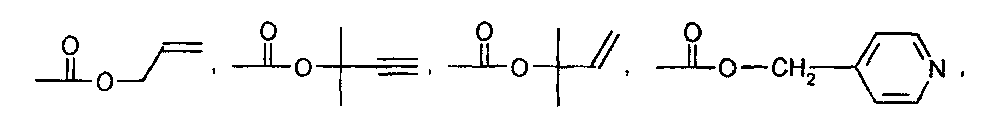 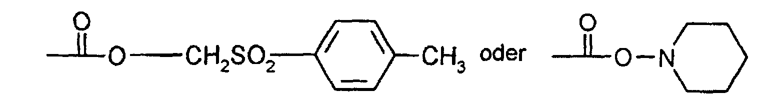 bedeuten.

Von besonderem Interesse ist auch das oben dargestellte Verfahren, worin latente Pigmente der Formel (IV) verwendet werden, worin L₁ und L₂ unabhängig voneinander C₂-C₁₈-Dialkylamino oder C₁-C₁₈-Cycloalkylamino bedeuten.

Die latenten Pigmente der Formel (III) und (IV) sind bekannte Substanzen, deren Synthese beispielsweise in EP-648770, EP-648817, Angewandte Chemie 68/4, 133-150 (1956) oder J. Org. Chem. 22, 127-132 (1957) angegeben ist. Sollten einige noch neu sein, so können sie in Analogie zu allgemein bekannten Methoden hergestellt werden. Anstatt eines latenten Pigments der Formel (IV), worin L₁ und L₂ in den Positionen 5/19 stehen, sind auch dessen Stellungsisomere, worin L₁ und L₂ beispielweise in den Positionen 5/6, 5/13, 5/26, 5/28, 6/13 oder 6/20 stehen, oder ein Gemisch von Stellungsisomeren möglich. Vielfach steht das bei der obengenannten Synthese entstehende Stellungsisomer nicht fest.

Bevorzugte Verbindungen der Formel (I) sind
i) 2,5-Dihydro-pyrrolo[3,4-c]pyrrole der Formel 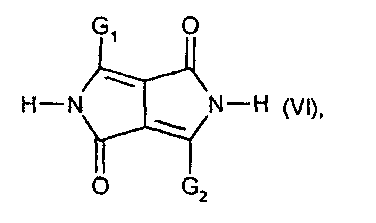 worin G₁ und G₂ unabhängig voneinander Gruppen der Formel 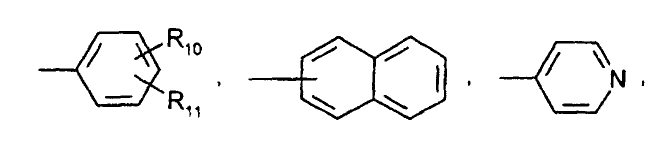 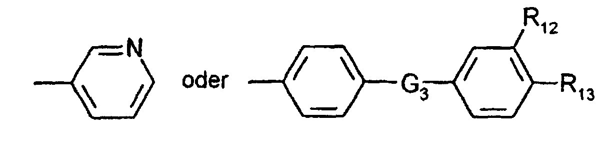 sind, worin G₃ -O-, -NR₁₄-, -N=N- oder -SO₂- bedeutet,
   - R₁₀ und R₁₁: unabhängig voneinander Wasserstoff, Chlor, Brom, C₁-C₄-Alkyl, C₁-C₆-Alkoxy, C₁-C₆-Alkylamino, CN oder Phenyl bedeuten,
   - R₁₂ und R₁₃: Wasserstoff und
   - R₁₄: Wasserstoff, Methyl oder Ethyl bedeuten;
ii) Perylencarbonsäureimide der Formel 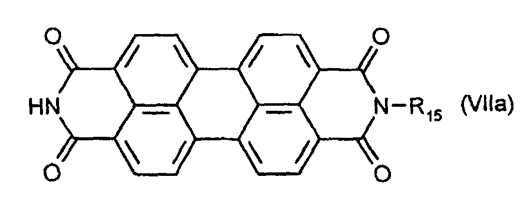 oder 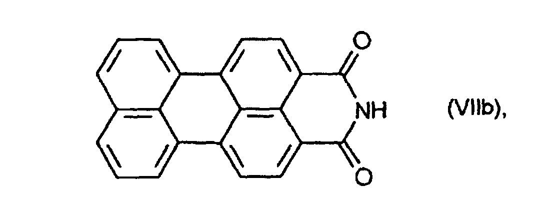
   - worin R₁₅: Wasserstoff, C₁-C₆-Alkyl, unsubstituiertes oder durch Halogen oder C₁-C₄-Alkyl substituiertes Phenyl, Benzyl oder Phenethyl bedeutet;
iii) Chinacridone der Formel 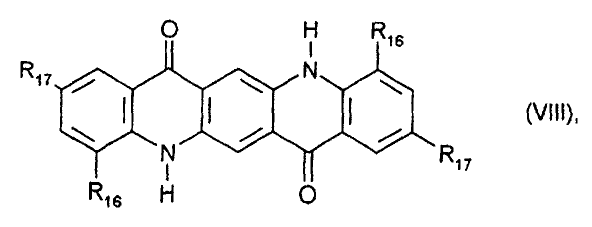
   - worin R₁₆ und R₁₇: unabhängig voneinander Wasserstoff, Halogen, C₁-C₁₈-Alkyl, C₁-C₄-Alkoxy oder Phenyl sind;
iv) Dioxazine der Formel 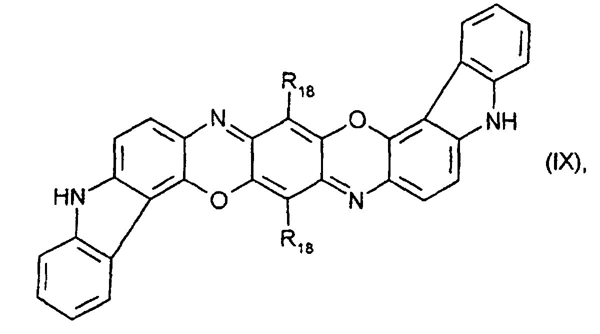
   - worin R₁₈: Wasserstoff, Halogen oder C₁-C₁₈-Alkyl ist;
v) Isoindoline der Formeln 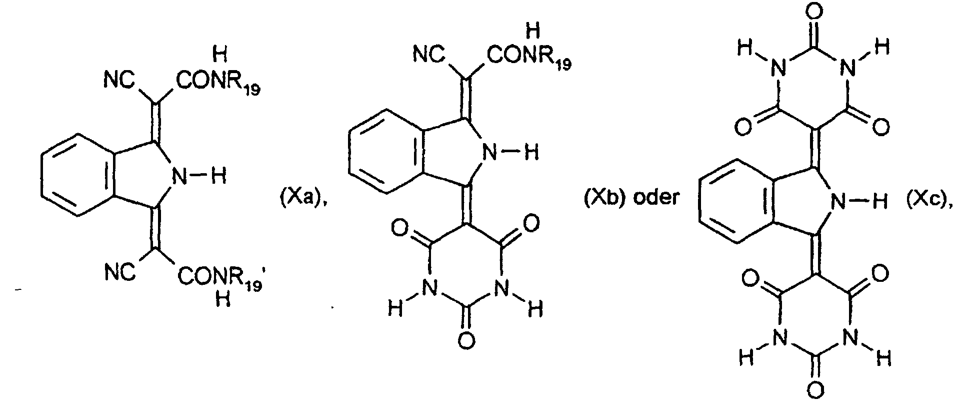
   - worin R₁₉: Wasserstoff, C₁-C₁₈-Alkyl, Benzyl oder eine Gruppe 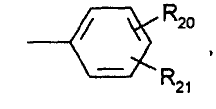 und
   - R_{19'}: eine Gruppe 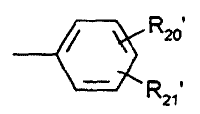 sind,
   wobei R₂₀, R₂₁, R₂₀' und R₂₁' unabhängig voneinander Wasserstoff, C₁-C₁₈-Alkyl, C₁-C₄-Alkoxy, Halogen oder Trifluormethyl bedeuten;
vi) Indigoderivate der Formel 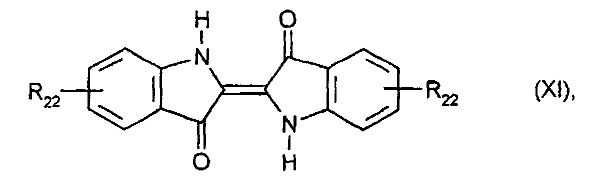
   - worin R₂₂: Wasserstoff, CN, C₁-C₄-Alkyl, C₁-C₄-Alkoxy oder Halogen bedeutet;
vii) Benzimidazolon-Azoverbindungen der Formel 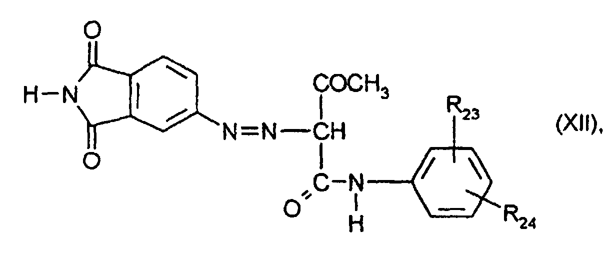 worin R₂₃ und R₂₄ unabhängig voneinander Wasserstoff, Halogen, C₁-C₄-Alkyl oder C₁-C₄-Alkoxy sind;
viii) anthrachinoide Verbindungen der Formel 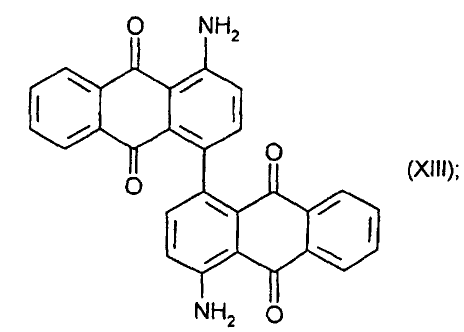 sowie
ix) Phthalocyanine der Formel 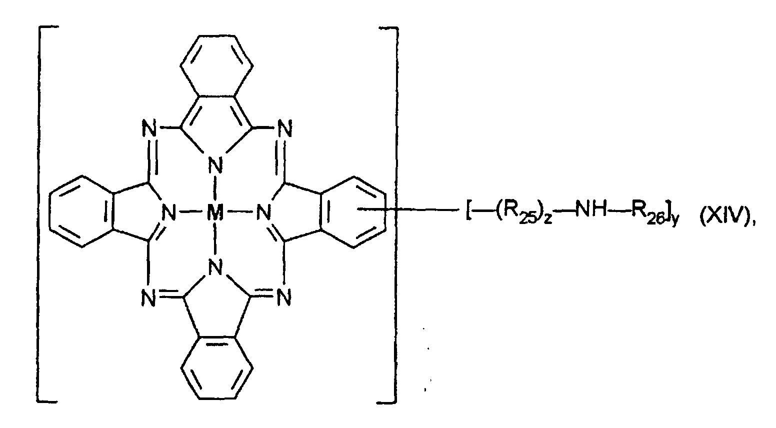 worin
   - M: H₂, Zn, Cu, Ni, Fe, TiO oder VO,
   - R₂₅: -CH(R₂₇)- oder -SO₂-,
   - R₂₆: Wasserstoff, C₁-C₄-Alkyl, -NHR₂₇, -NHCOR₂₈, -COR₂₈
   oder 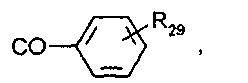
   - R₂₇: Wasserstoff oder C₁-C₄-Alkyl,
   - R₂₈: C₁-C₄-Alkyl,
   - R₂₉: Wasserstoff, Halogen, C₁-C₄-Alkyl oder C₁-C₄-Alkoxy,
   - z: 0 oder 1 und
   - y: eine Zahl von 1 bis 4 bedeuten.
   Bevorzugte Verbindungen der Formel (II) sind
x) Phthalocyanine der Formel 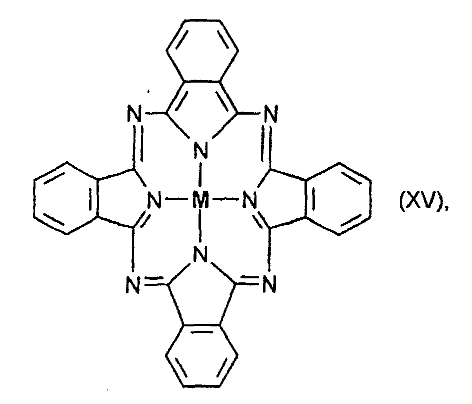
   - worin: M H₂, Zn, Cu, Ni, Fe, TiO oder VO bedeutet.

Unter den Pyrrolopyrrolen sind jene der Formel (Vl), worin G₁ und G₂ identisch sind, insbesondere jene, worin G₁ und G₂ gleiche Gruppen der Formel 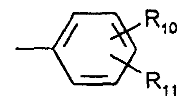 sind, worin R₁₀ und R₁₁ unabhängig voneinander Wasserstoff, Methyl, tert.-Butyl, Chlor, Brom, CN oder Phenyl bedeuten, ganz besonders jene, worin R₁₁ Wasserstoff ist, besonders bevorzugt.

Unter den Phthalocyaninen sind jene der Formel (XIV), worin M H₂, Cu oder Zn, R₂₅ -CH₂oder -SO₂-, R₂₆ Wasserstoff, -NHCOCH₃ oder Benzoyl, R₂₇ Wasserstoff und z die Zahl 1 bedeuten, sowie insbesondere jene der Formel (XV), worin M H₂, Cu oder Zn bedeutet, besonders bevorzugt.

Das zu beschichtende Substrat kann inbezug auf seine chemische Zusammensetzung und auf seine Form beliebig sein. Beispielsweise kann es sich dabei um Metalle, Metalloxyde, Kunststoffe, Nichtmetalle oder um komposite Materialien handeln, wie Glas, Porzellan, blanker, grundierter oder lackierter Stahl, Aluminium, Silicium, Indium/Zinn-Oxyd, Galliumarsenid, Polyvinylchlorid, Polyethylenterephthalat, Polypropylen, Polyamid, Polyacrylat, Polystyrol, sowie deren Gemische, Legierungen oder Copolymere. Es kann sich dabei um beliebige Waren und Gegenstände handeln, beispielsweise in Form von Platten, Blechen, Folien, Röhren, Fasern, Geweben, Flaschen, Laminate oder Wafern.

Handelt es sich beim Substratmaterial teilweise oder ganz um einen Kunststoff, so kann es sich dabei um ein beliebiges hochmolekulares organisches Material handeln, zum Beispiel Vinylpolymere, wie Polystyrol, Poly-α-methylstyrol, Poly-p-methylstyrol, Poly-p-hydroxystyrol, Poly-p-hydroxyphenylstyrol, Poly(methylacrylat) und Poly(acrylamid) sowie die entsprechenden Methacrylverbindungen, Poly(methylmaleat), Poly(acrylnitril), Poly(methacrylnitril), Poly(vinylchlorid), Poly(vinylfluorid), Poly(vinylidenchlorid), Poly(vinylidenfluorid), Poly(vinylacetat), Poly(methylvinylether) und Poly(butylvinylether); Novolake abgeleitet von C₁-C₆-Aldehyden, wie Formaldehyd und Acetaldehyd, und einem zweikemigen oder vorzugsweise einkernigen Phenol, das gegebenenfalls mit einer oder zwei C₁-C₉-Alkylgruppen, einem oder zwei Halogenatomen oder einem Phenylring substituiert ist, wie o-, m- oder p-Kresol, Xylol, p-tert-Butylphenol, p-Chlorphenol oder p-Phenylphenol, o-, m- oder p-Nonylphenol, oder einer Verbindung mit mehr als einer phenolischen Gruppe, wie Resorcin, Bis-(4-hydroxyphenyl)methan oder 2,2-Bis-(4-hydroxyphenyl)propan; von Maleinimid und/oder Maleinanhydrid abgeleitete Polymere, wie Copolymere von Maleinanhydrid und Styrol; Poly(vinylpyrrolidon), Biopolymere und deren Derivate, wie Cellulose, Stärke, Chitin, Chitosan, Gelatine, Zein, Ethylcellulose, Nitrocellulose, Celluloseacetat und Cellulosebutyrat; natürliche Harze und Kunstharze, wie Gummi, Casein, Silikon und Silikonharze, ABS, Harnstoff- und Melamin-Formaldehydharze, Alkydharze, Phenolharze, Polyamide, Polyimide, Polyamid/imide, Polysulfone, Polyethersulfone, Polyphenylenoxide, Polyurethane, Polyharnstoffe, Polycarbonate, Polyarylene, Polyarylensulfide, Polyepoxide, Polyolefine und Polyalkadiene.

Bevorzugte hochmolekulare organische Materialien sind Celluloseether und -ester, wie Ethylcellulose, Nitrocellulose, Celluloseacetat oder Cellulosebutyrat, natürliche Harze oder Kunstharze, wie Polymerisations- oder Kondensationsharze, wie Aminoplaste, insbesondere Harnstoff- und Melamin-Formaldehydharze, Alkydharze, Phenoplaste, Polycarbonate, Polyester, Polyolefine, Polystyrol, Polyvinylchlorid, Polyamide, Polyurethane, ABS, Polyphenylenoxide, Silikon und Silikonharze, sowie deren Gemische.

Zur Beschichtung wird ein latentes Pigment einfach geschmolzen, oder bevorzugt in einen geeigneten Lösemittel gelöst. Auch Gemische latenter Pigmente können benützt werden, wobei die einzelnen Komponenten zusammen oder nacheinander geschmolzen oder in einen geeigneten Lösemittel gelöst werden, wodurch tiefere Mischschmelzpunkte bzw. höhere Konzentrationen möglich sind. Vorzugsweise wird das latente Pigment unter Rühren bei einer etwas erhöhten Temperatur gelöst, zweckmässig zwischen 30°C und dem Siedepunkt des Lösemittels.

Als Lösemittel eignen sich alle üblichen Lösemittel, wie unter anderen Kohlenwasserstoffe, Alkohole, Amide, Nitrile, Nitroverbindungen, N-Heterozyklen, Ether, Ketone und Ester, welche gegebenenfalls auch einfach oder mehrfach ungesättigt oder chloriert sein können, zum Beispiel Methanol, Ethanol, Isopropanol, Diethylether, Aceton, Methylethylketon, 1,2-Dimethoxyethan, 1,2-Diethoxyethan, 2-Methoxyethanol, Essigsäureethylester, Tetrahydrofuran, Dioxan, Acetonitril, Benzonitril, Nitrobenzol, N,N-Dimethylforrnamid, N,N-Dimethylacetamid, Dimethylsulfoxyd, N-Methylpyrrolidon, Pyridin, Picolin, Chinolin, Trichlorethan, Benzol, Toluol, Xylol, Anisol oder Chlorbenzol. Weitere Beispiele von Lösemitteln sind in vielen Tabellen- und Referenzwerken beschrieben. Anstatt eines einzigen Lösemittels können auch Gemische mehrerer Lösemitteln eingesetzt werden.

Bevorzugt sind solche Lösemittel, welche das zu beschichtende Substrat nicht oder nur sehr langsam lösen, und welche einen Siedepunkt zwischen 40°C und 170°C haben, insbesondere aromatische Kohlenwasserstoffe, Alkohole, Ether, Ketone und Ester. Besonders bevorzugt sind Toluol, Methanol, Ethanol, Isopropanol, 1,2-Dimethoxyethan, 1,2-Diethoxyethan, Aceton, Methylethylketon, Essigsäureethylester, Tetrahydrofuran und Dioxan, sowie deren Gemische.

Die bevorzugte Konzentration der latenten Pigmente in einem Lösemittel beträgt zirka 80-99% der Sättigungskonzentration, wobei in einigen Fällen auch übersättigte Lösungen ohne vorzeitige Ausfällung des Solvats gehandhabt werden können. Bei vielen latenten Pigmenten der Formeln (III) oder (IV) liegt die optimale Konzentration um ∼0,1-50 Gew.-%, oft um 1-20 Gew.-%.

Bei der Wahl des Lösemittels und der optimalen Konzentration sollten die Erfordernisse der gewählten Beschichtungstechnik an die physikalischen Eigenschaften der Lösung, zum Beispiel inbezug auf spezifisches Gewicht, Dampfdruck sowie insbesondere Viskosität, berücksichtigt werden.

Enthält die Lösung ungelöste Partikel, beispielsweise Staub, Pigmentteilchen oder andere Verunreinigungen, oder benützt man zur Beschichtung aufwendige Techniken, zum Beispiel Aufschleudern, so empfiehlt es sich, diese Lösung vor dem weiteren Gebrauch zu filtrieren, besonders vorteilhaft durch einen Filter mit sehr feinen Poren. Empfehlenswert ist auch eine staubfreie Umgebung, beispielsweise ein Reinluftraum.

Die Beschichtung kann mit einer beliebigen bekannten Technik erfolgen, wie durch Eintauchen, Spritzen, Bedrucken, Giessen, Aufziehen oder Aufschleudem. Solche übliche Techniken sowie deren Vorteile und Nachteile sind in sehr vielen Publikationen bestens beschrieben. Besonders vorteilhafte, homogene Beschichtungen werden durch Aufschleudem erzielt.

Bei der Beschichtung kann das Substrat vollständig, oder bevorzugt auf nur einem Teil seiner Oberfläche beschichtet werden, wodurch farbige Muster entstehen. Einige Beschichtungstechniken ergeben dabei Muster von selbst, wie beim Bedrucken je nach Muster der Druckplatte, bei anderen Beschichtungstechniken kann man sich üblicher Hilfsmittel bedienen, wie Verwendung einer Schablone beim Spritzen. Bevorzugt wird das Substrat in Schritt (a) des erfindungsgemässen Verfahrens auf nur einem Teil seiner Oberfläche beschichtet.

Zweckmässig wird nach der Beschichtung das Lösemittel teilweise oder vollständig entfernt, indem es zum Beispiel bei 0,1-1,0 bar verdampfen gelassen wird. Bevorzugt wird das Lösemittel unter einem Abzug bei einer Temperatur 1-50°C unterhalb des Flammpunktes des Lösemittels und einem Druck von 0,7-1,0 bar auf eine Restmenge ≤ 50 Gew.-%, bezogen auf das latente Pigment, verdampfen gelassen.

Anschliessend wird das auf dem Substrat beschichtete latente Pigment partiell oder vollständig in dessen unlösliche Pigmentform überführt. Dabei müssen die Gruppen D₃ und gegebenenfalls D₄ in Formel (III), beziehungsweise L₁ und L₂ in Formel (IV), abgespalten werden. Bei latenten Pigmenten der Formel (III) erfolgt dieser Schritt mit Leichtigkeit, wobei die Gruppen D₃ und gegebenenfalls D₄ fragmentieren und am Chromophorrest durch Wasserstoffatome ersetzt werden. Bei latenten Pigmenten der Formel (IV) erfolgt dieser Schritt ebenfalls mit Leichtigkeit, wobei jedoch L₁ und L₂ keine Wasserstoffatome hinterlassen (d.h. formal findet eine Elimination statt).

Die Überführung (b) der latenten Pigmente in deren unlösliche Form kann auf einfachste Weise erzielt werden, sei es durch thermische (Aufheizen auf Temperaturen zum Beispiel zwischen 50 und 400°C, bevorzugt auf 100 bis 200°C, insbesondere durch Annäherung einer Heizquelle oder Bestrahlung mit Infrarotlicht), photolytische (Bestrahlung zum Beispiel mit UV-Licht der Wellenlänge ≤ 375 nm) oder chemische Behandlung (Aussetzen dem Dampf einer organischen oder anorganischen Brønsted- oder Lewis-Säure oder einer Base, wie Salzsäure, Trifluoressigsäure, Bortrifluorid, Ammoniak oder 4-Dimethylaminopyridin). Die erwähnten Umwandlungsmethoden können auch kombiniert werden, wie beispielsweise bei der Bestrahlung mit einem nicht fokussierten oder besonders bevorzugt mit einem fokussierten Laserstrahl, zum Beispiel derjenige eines Argonionenlasers mit Linien bei 488 und 514 nm, derjenige eines CO₂-Lasers mit Linien bei ∼10 µm oder derjenige eines Nd:YAG-Lasers mit Hauptlinie bei 1064 nm, gegebenenfalls mit Frequenzverdoppelung.

Durch die Wahl der Bedingungen zur Überführung (b) der latenten Pigmente in dessen unlösliche Form kann die Struktur der Pigmentbeschichtung, insbesondere inbezug auf die Partikelgrösse der Pigmente, reproduzierbar beeinflusst werden. Beispielsweise können unter verschiedenen Bedingungen entweder feine (Durchmesser ≤ 0.1 µm), mittlere (Durchmesser ∼0.1-1 µm) oder auch grobe (Durchmesser ≥ 1 µm) Pigmentpartikel erhalten werden. In vielen Fällen hat es sich als günstig erwiesen, das Substrat selbst bei der Umwandlung auf eine leicht erhöhte Temperatur (∼ 50-80°C) zu erwärmen.

Bei Schritt (b) des erfindungsgemässen Verfahrens kann man das latente Pigment vollflächig und quantitativ in dessen unlösliche Pigmentform überführen. Bevorzugt erfolgt Schritt (b) jedoch nur auf ausgewählten Gebieten, wobei sehr leicht strukturierte Farbmuster erzielt werden können. Die Auflösung solcher Strukturierungen hängt von der Dimension des zur Umwandlung benützten Mittels ab, also beispielsweise von den Dimensionen eines als Heizquelle benützten Thermoelementen oder vom Durchmesser eines als Bestrahlungsmittel benützten fokussierten oder nicht fokussierten Lichtstrahles.

Benützt man zur selektiven Umwandlung einen Laserstrahl, so sind strukturierte Farbmuster mit besonders hohen Auflösungen bei den erzielten strukturierten Farbmustem möglich, zum Beispiel 1 µm bis 1 mm, bevorzugt 5-200 µm, besonders bevorzugt 10-50 µm. Unter Auflösung versteht man dabei der kleinstmögliche Intervall zwischen aufeinanderfolgende Linien, die durch leere Linien getrennt sind. Mit Laserstrahlen und Thermoelementen können strukturierte Farbmuster auf einfache und schnelle Weise erzielt werden, beispielsweise mit einem Laserbeschriftungsgerät oder analog den bekannten Technologien von Laser- oder Thermodruckern. Solche Vorgänge werden bevorzugt mit einer programmierten Steuerung, besonders bevorzugt vollautomatisch gelenkt, zweckmässig mit einer speicherprogrammierten Steuerung oder einem Computer mit festen oder variablen Daten. Die strukturierten Farbmuster können beliebig sein, wie aufeinanderfolgende Bildpunkte (pixels), beispielsweise in einer Spirale angeordnet, Streifenkodierungen, Buchstaben oder Schriftzüge, sowie geometrische oder künstlerisch gestaltete Formen, beispielsweise Markenzeichen.

Falls man keine hohe Auflösung braucht, beispielsweise für Dekorationszwecke, kann man auch solchermassen vorgehen, dass man die wie zuvor beschrieben bei Schritt (a) erzeugten farbigen Muster bei Schritt (b) ganzflächig in die unlösliche Pigmentform überführt. Man kann aber auch bei Schritt (a) den groben Umriss des erwünschten farbigen Musters erzeugen, und danach bei Schritt (b) das erwünschte farbige Muster hochaufgelöst in die unlösliche Pigmentform überführen. Man kann ebenfalls gut aufgelöste Strukturierungen erzeugen, indem man durch eine feste oder programmierbare Maske bestrahlt, beispielsweise durch eine Schablone oder einen Flüssigkristallschirm.

Erfolgt Schritt (b) nur auf ausgewählte Gebiete, so verbleiben neben den die Pigmentbeschichtung aufweisenden Gebieten auch solche mit unverändertem latenten Pigment. Verwendet man im Vergleich zur Stabilität des latenten Pigmentes milde Bedingungen zu dessen Überführung in die unlösliche Pigmentform, so kann auch manchmal ein Teil des latenten Pigmentes nur partiell umgewandelt werden, so dass die Pigmentbeschichtung noch lösliche Anteile beinhaltet. In beiden Fällen erfolgt bevorzugt im Anschluss auf Schritte (a) und (b) zusätzlich (c) die Entfernung des übriggebliebenen latenten Pigmentes.

Die Entfernung des übriggebliebenen latenten Pigmentes erfolgt bevorzugt mit Hilfe eines Lösemittels, zum Beispiel durch Tauchen, beispielweise im Gegenstrom, oder durch Spülen, beispielsweise mit einer Druckspritze oder im kondensierenden Dampf, gegebenenfalls mit mechanischer Hilfe, wie Bürsten, Schwenken oder Ultraschall. Bevorzugt sind die gleichen Lösemittel wie für die Beschichtung. Die Lösung des zurückgewonnenen übriggeblienenen latenten Pigmentes kann wiederverwertet werden, gegebenenfalls nach deren Reinigung, beispielsweise durch Filtration.

Nachdem eine Pigmentbeschichtung nach dem erfindungsgemässen Verfahren auf ein Substrat angebracht wurde, kann auf gleiche Weise eine weitere Pigmentbeschichtung angebracht werden, so dass das Substrat mehrere Pigmentbeschichtungen aufweist. Dieser Vorgang kann im Prinzip beliebig oft wiederholt werden.

Die Erfindung betrifft deshalb auch ein Verfahren, worin mehrere Pigmentbeschichtungen an der Substratoberfläche angebracht werden, durch aufeinanderfolgendes Anbringen dieser Pigmentbeschichtungen unter jeweiliger Wiederholung der Schritte (a) bis (c) für jede Pigmentbeschichtung.

Mit der erfindungsgemässen Methode sind überraschend sehr homogene, gleichmässige und transparente Beschichtungen mit hohen Echtheiten möglich. Am äusseren Rand eines Farbmusters gibt es eine schmale Zone, deren Breite von der Auflösung der Umwandlungsmethode und der Thermokonduktivität des Substrates abhängt, beispielsweise ≤ 5-10 µm bei einem gut fokussierten Laser. Die Gleichmässigkeit der Pigmentbeschichtungs-Schichtdicke entspricht derjenigen des latenten Pigmentes; je nach Beschichtungsmethode variert sie um weniger als ± 25%, meist weniger als ± 10%, bei präzisen Beschichtungstechniken wie Aufschleudem sogar bis unter ± 1%. Das Reflektionsspektrum ist qualitativ an allen Stellen deckungsgleich, quantitativ ist es einzig von der Schichtdicke abhängig.

Davon abgesehen ist die Pigmentbeschichtung an den Stellen, wo das latente Pigment in dessen unlösliche Pigmentform überführt wurde, im Wesentlichen gleichmässig. Insbesondere sind sehr gleichmässig verteilte, feine Pigmentpartikel (beispielsweise solche mit einem Durchmesser ≤ 0,1 µm) möglich, so dass sich hervorragend transparente und koloristisch brillante Färbungen ergeben. An den Stellen, wo keine Behandlung zwecks Überführung des latenten Pigmentes in dessen unlösliche Pigmentform stattfand, fehlt die Pigmentbeschichtung gänzlich, so dass dort die optische Transparenz derjenigen des blanken Substrates entspricht.

Die Erfindung betrifft deshalb auch ein Material enthaltend ein Substrat, an dessen Oberfläche mindestens eine aus einem oder mehreren Pigmenten der Formel (I) oder (II) gemäss Anspruch 1 bestehende Pigmentbeschichtung angebracht ist, dadurch gekennzeichnet, dass diese Pigmentbeschichtung an einem Teil der Substratoberfläche im Wesentlichen gleichmässig, und am Rest der Substratoberfläche nicht vorhanden ist.

Zum Schutz gegen äussere Einflüsse ist es möglich, das erfindungsgemässe Material zusätzlich mit einer Schutzschicht zu versehen, beispielsweise mit einem aus einem der obengenannten Kunststoffe bestehenden Lack, welcher gegebenenfalls Additive zur weiteren Verbesserung der Pigmenteigenschaften enthalten kann, zum Beispiel Lichtstabilisatoren, wie gehinderte Amine, oder UV-Absorber, wie Hydroxybenzophenone, Hydroxyphenylbenzotriazole, Hydroxyphenyltriazine oder Oxalanilide. Bei mehreren Pigmentbeschichtungen ist es auch möglich, eine Schutzschicht zwischen den einzelnen Beschichtungen vorzunehmen, so dass die nächste Pigmentbeschichtung nicht unmittelbar auf das Substrat, sondern auf diese Zwischenschicht angebracht wird.

Das erfindungsgemässe Material kann eine oder mehrere Pigmentbeschichtungen aufweisen. Werden auf ein Substrat mehrere Pigmentbeschichtungen angebracht, so sind Beschichtungen aus Pigmenten der Formeln (I) oder (II) oder Gemische davon bevorzugt, welche unterschiedliche Absorptionen, wie breite bzw. scharfe Absorptionsbande, oder unterschiedliche Absorptionsmaxima, wie 450 nm bzw. 550 nm oder 650 nm aufweisen. Bevorzugt bildet dabei jede der Pigmentbeschichtungen ein eigenes, mit den Mustern der anderen Pigmentbeschichtungen nicht deckungsgleiches Muster, so dass mehrfarbige Muster entstehen. Die Erfindung betrifft deshalb auch ein Material, worin auf dem Substrat mehrere Pigmentbeschichtungen angebracht sind, welche nicht deckungsgleiche Muster bilden und unterschiedliche Absorptionen und/oder unterschiedliche Absorptionsmaxima aufweisen.

Besonders bevorzugt ist ein solches Material, worin die Pigmentbeschichtungen aus derartigen Pigmenten bestehen und derart angeordnet sind, dass sich blaue, grüne und rote Gebiete ergeben, und worin das Substrat transparent ist.

Anstatt mehrerer verschiedenfarbiger Pigmentbeschichtungen sind auch mehrere, gleiche oder gleichfarbige Pigmente enthaltende Pigmentbeschichtungen unterschiedlicher Schichtdicken möglich. Überlappen diese, so ergeben sich dann je nach Art der Überlappung Muster verschiedener Farbintensitäten. Bevorzugt ist es, dass die Totalabsorption, die sich durch die Summe der Intensitäten aller sich überlappenden Pigmentbeschichtungen ergibt, an jedem mindestens eine Pigmentbeschichtung aufweisenden Bildpunkt ein Mehrfaches des Wertes der kleinsten Absorption darstellt. Die kleinste Absorption ist diejenige der dünnsten Pigmentbeschichtung und kommt an Bildpunkten vor, wo diese dünnste Beschichtung mit keiner anderen Pigmentbeschichtung überlappt. Vorteilhaft sind Pigmentbeschichtungen, deren relativen Schichtdicke (bzw. deren relativen Absorptionen) im Verhältnis 1, 2, 4, 8 ... 2ⁿ stehen, womit auch die Zwischenwerte durch gezielte Überlappung möglich sind. Unterschiedliche Schichtdicken können auch mit verschiedenfarbigen Pigmentbeschichtungen kombiniert werden.

Die erfindungsgemässen Materialien können zur dekorativen Färbung verwendet werden. Bevorzugt ist aber deren Verwendung zur permanenten Speicherung von digitaler Information, sowie besonders bevorzugt deren Verwendung als Farbfilter, wozu sich insbesondere die bereits erwähnten Materialien eignen, die aus einem transparenten Substrat sowie blaue, grüne und rote Pigmentbeschichtungen aufweisenden Gebieten bestehen. Bei der Verwendung als Farbfilter, beziehungsweise zur Speicherung von digitaler Information, sind insbesondere die hohe Auflösung, die hohe Transparenz, die im Vergleich mit entsprechenden löslichen Farbstoffen ausgezeichneten Pigmentechtheiten sowie die im Vergleich mit Massenfärbung wesentlich dünneren Farbschichten vorteilhaft.

Wird das erfindungsgemässe Material zur Speicherung von digitaler Information verwendet, so kann letztere durch Bestrahlen des Materials mit einer Lichtquelle, beispielsweise mit einem fokussierten Laserstrahl aufgezeichnet, und Messung der Intensität des reflektierten oder des transmittierten, bevorzugt deutlich energieärmeren Lichtstrahles abgelesen werden. Durch die Homogenität und die Stabilität der Pigmentbeschichtung sind häufige, präzise Ablesevorgänge ohne Abnahme der Lesequalität möglich. Mehrere gleichartige, überlappende Pigmentbeschichtungen unterschiedlicher Dicke aufweisende Materialien ergeben Signale verschiedener Intensitäten, so dass pro Bildpunkt mehrere Bits gespeichert werden können. Die Erfindung betrifft deshalb auch ein Verfahren zum Ablesen von auf einem erfindungsgemässen Material gespeicherter digitaler Information durch Bestrahlen mit einer Lichtquelle und Messung der Intensität des reflektierten oder des transmittierten Lichtstrahles.

Die folgenden Beispiele erläutern die Erfindung:
Beispiel 1 Eine Lösung von 100 mg N,N'-Bis(tert.-butoxycarbonyl)-3,6-diphenyl-1,4-diketopyrrolo[3,4c]-pyrrol der Formel (XVIa) 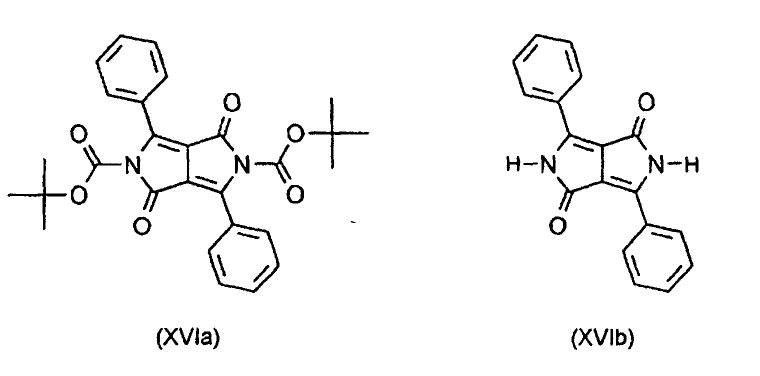 in 2 ml Dioxan wird bei 1000 U/Min auf einen 38×26×1 mm Glasträger aufgeschleudert. Die entstandene Schicht wird bei 90°C während 1 Min getrocknet. Die gelbe Schicht hat eine Dicke von ∼360 nm und die optische Dichte bei λₘₐₓ = 430 nm beträgt 0,90. Der beschichtete Träger wird dann auf einer auf 200°C vorgeheizten Platte während 5 Min erhitzt, wobei eine Farbänderung zur charakteristischen roten Farbe von 2,5-Dihydro-3,6-diphenyl-1,4-diketo-pyrrolo[3,4c]-pyrrol der Formel (XVlb) stattfindet. Die entstandene Schicht hat eine Dicke von ∼250 nm und die optische Dichte bei λₘₐₓ = 540 nm beträgt 0,80. Das nach thermischer Behandlung erhaltene Absorptionsspektrum ist identisch mit demjenigen von 2,5-Dihydro-3,6-diphenyl-1,4-diketo-pyrrolo[3,4c]-pyrrol. Die Schicht zeigt hervorragende Transparenz und Homogenität.
Beispiel 2 Eine Lösung von 100 mg Leucophthalocyanin der Formel (XVIIa) 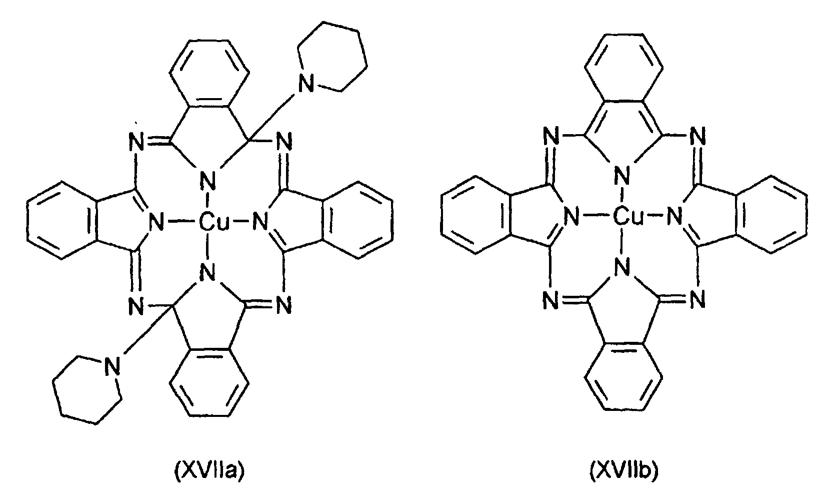 in 2 ml Dioxan wird bei 1000 U/Min auf einen 38×26×1 mm Glasträger aufgeschleudert. Die entstandene Schicht wird bei 90°C während 1 Min getrocknet. Die leicht gelbliche Schicht hat eine Dicke von ∼320 nm und die optische Dichte bei λₘₐₓ = 311 nm beträgt 1,05. Der beschichtete Träger wird dann auf einer auf 200°C vorgeheizten Platte während 5 Min erhitzt, wobei eine Farbänderung zur charakteristischen blauen Farbe von Kupferphthalocyanin der Formel (XVllb) stattfindet. Die entstandene Schicht hat eine Dicke von ∼300 nm und die optische Dichte bei λₘₐₓ = 620 nm beträgt 0,87. Das nach thermischer Behandlung erhaltene Absorptionsspektrum ist identisch mit demjenigen von Kupferphthalocyanin. Die Schicht zeigt hervorragende Transparenz und Homogenität.
Beispiel 3 Eine Lösung von 100 mg N,N'-Bis(tert.-butoxycarbonyl)-3,6-diphenyl-1,4-diketopyrrolo[3,4c]-pyrrol der Formel (XVla) in 2 ml Dioxan wird bei 1000 U/Min auf einen 38×26×1 mm Glasträger aufgeschleudert. Die entstandene Schicht wird bei 90°C während 2 Min getrocknet. Die gelbe Schicht hat eine Dicke von ∼360 nm und die optische Dichte bei λₘₐₓ = 430 nm beträgt 0,90. Der beschichtete Träger wird dann bei 25°C während 30 Min in Chlorwasserstoff-Dampf gelagert, wobei eine Farbänderung zur charakteristischen roten Farbe von 2,5-Dihydro-3,6-diphenyl-1,4-diketo-pyrrolo[3,4c]-pyrrol der Formel (XVIb) stattfindet. Die entstandene Schicht hat eine Dicke von ∼250 nm und die optische Dichte bei λₘₐₓ = 540 nm beträgt 0,80. Das nach Säurebehandlung erhaltene Absorptionsspektrum ist identisch mit demjenigen von 2,5-Dihydro-3,6-diphenyl-1,4-diketo-pyrrolo[3,4c]-pyrrol. Die Schicht zeigt hervorragende Transparenz und Homogenität.
Beispiel 4 Eine Lösung von 100 mg Dioxazin-Derivat der Formel (XVIIIa) 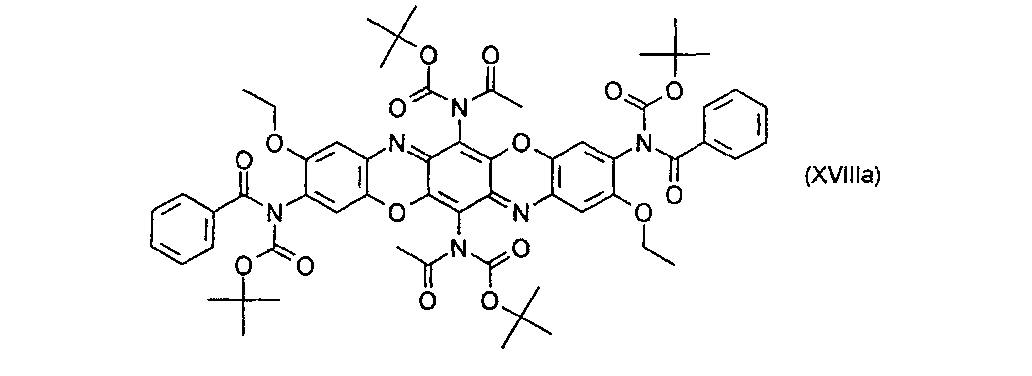 in 2 ml Dioxan wird bei 1000 U/Min auf einen 38×26×1 mm Glasträger aufgeschleudert. Die entstandene Schicht wird bei 90°C während 1 Min getrocknet. Die rote Schicht hat eine Dicke von ∼120 nm und die optische Dichte bei λₘₐₓ = 561 nm beträgt 0,56. Der beschichtete Träger wird dann auf einer auf 200°C vorgeheizten Platte während 5 Min erhitzt, wobei eine Farbänderung zur charakteristischen violetten Farbe vom Dioxazin-Pigment der Formel (XVlllb = C.I. Pigment Violet 37) stattfindet. 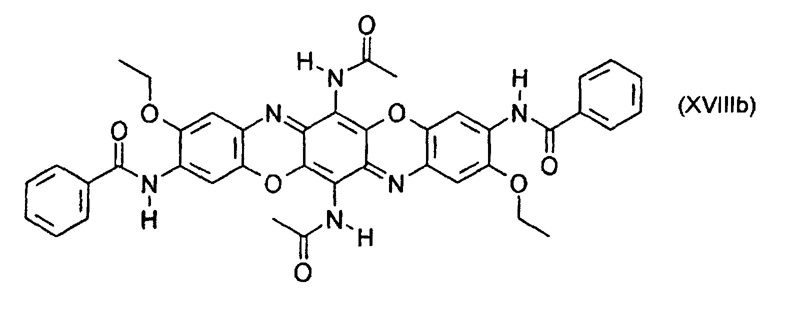 Die entstandene Schicht hat eine Dicke von ∼80 nm und die optische Dichte bei λₘₐₓ = 553 nm beträgt 0,43. Das nach thermischer Behandlung erhaltene Absorptionsspektrum ist identisch mit demjenigen von C.l. Pigment Violet 37. Die Schicht zeigt hervorragende Transparenz und Homogenität.
Beispiel 5 Eine Lösung von 100 mg der Formel (XVllla) in 2 ml Dioxan wird bei 1000 U/Min auf einen 38×26×1 mm Glasträger aufgeschleudert. Die entstandene Schicht wird bei 90°C während 1 Min getrocknet. Die gelbe Schicht hat eine Dicke von ∼120 nm und die optische Dichte bei λₘₐₓ = 561 nm beträgt 0,56. Der beschichtete Träger wird dann mit dem fokussierten Strahl eines Ar-lonenlasers (sichtbar, alle Linien; primär 488 und 514 nm) belichtet. Dabei wird der Laserstrahl über einen beweglichen Spiegel (Laserbeschriftungsgerät) unter Computersteuerung mit vorgegebener Geschwindigkeit über die Schicht geführt. Bei einer Laserleistung von 1,5 W, wobei die Schreibgeschwindigkeit zwischen 75 und 400 mm/s eingestellt wird, tritt an den beschrifteten Stellen eine Farbänderung zur charakteristischen violetten Farbe des Dioxazin-Pigments der Formel (XVlllb = C.l. Pigment Violet 37). Der Träger wird nachträglich mit 20 ml Diethylether gewaschen, wobei nur die beschrifteten (unlöslichen) Strukturen in Relief auf dem Glasträger bleiben. Die Auflösung ist ≤ 50 µm.
Beispiel 6 Es wird so wie in Beispiel 5 verfahren, jedoch wird als Träger ein 50×75×2 mm grosses Stück Polycarbonat (®Makrolon, Bayer) verwendet, und die Beschriftung erfolgt bei einer Leistung von 1,0 W. Die Resultate sind mit denen von Beispiel 5 vergleichbar.
Beispiel 7 Es wird so wie in Beispiel 1 verfahren, jedoch wird mit dem fokussierten Strahl eines Ar-lonenlasers mit UV-Spiegel (UV, alle Linien; primär 351 und 364 nm) bei einer Leistung von 0,4 W belichtet. Die Resultate sind mit denen von Beispiel 5 vergleichbar.
Beispiel 8 Wie in Beispiel 1 wird eine Lösung von 100 mg N,N'-Bis(tert.-butoxycarbonyl)-3,6-Biphenyl-1,4-diketo-pyrrolo[3,4c]-pyrrol der Formel (XVIa) in 2 ml Dioxan bei 1000 U/Min auf einen 38×26×1 mm Glasträger aufgeschleudert. Die entstandene Schicht wird bei 90°C während 1 Min getrocknet. Die gelbe Schicht hat eine Dicke von ∼360 nm und die optische Dichte bei λₘₐₓ = 430 nm beträgt 0,90. Der beschichtete Träger wird dann mit dem fokussierten Strahl eines Ar-lonenlasers (sichtbar, alle Linien; primär 488 und 514 nm) belichtet. Bei einer Laserleistung von 2,5 W und einer Schreibgeschwindigkeit von 75 mm/s tritt an den beschrifteten Stellen eine Farbänderung zur charakteristischen roten Farbe des Diketopyrrolopyrrol-Pigments der Formel (XVIb).
Beispiel 9 Es wird so wie in Beispiel 8 verfahren, jedoch wird mit dem fokussierten Strahl eines Ar-lonenlasers mit UV-Spiegel (UV, alle Linien; primär 351 und 364 nm) bei einer Leistung von 0,8 W und einer Schreibgeschwindigkeit von 5 mm/s belichtet. Die Resultate sind mit denen von Beispiel 8 vergleichbar.
Beispiel 10 Wie in Beispiel 2 wird eine Lösung von 100 mg Leucophthalocyanin der Formel (XVlla) in 2 ml Dioxan bei 1000 U/Min auf einen 38×26×1 mm Glasträger aufgeschleudert. Die entstandene Schicht wird bei 90°C während 1 Min getrocknet. Die leicht gelbliche Schicht hat eine Dicke von ∼320 nm und die optische Dichte bei λₘₐₓ = 311 nm beträgt 1,05. Der beschichtete Träger wird dann mit dem fokussierten Strahl eines Ar-lonenlasers (sichtbar, alle Linien; primär 488 und 514 nm) belichtet. Bei einer Laserleistung von 2,5 W und einer Schreibgeschwindigkeit zwischen 75 und 200 mm/s tritt an den beschrifteten Stellen eine Farbänderung zur charakteristischen blauen Farbe des Phthalocyanin-Pigments der Formel (XVIIb).
Beispiel 11 Es wird so wie in Beispiel 10 verfahren, jedoch wird mit dem fokussierten Strahl eines Ar-lonenlasers mit UV-Spiegel (UV, alle Linien; primär 351 und 364 nm) bei einer Leistung von 0,8 W und einer Schreibgeschwindigkeit zwischen 5 und 60 mm/s belichtet. Die Resultate sind mit denen von Beispiel 10 vergleichbar.
Beispiel 12 Es wird so wie in Beispiel 10 verfahren, jedoch wird anstelle des Produkts der Formel (XVlla) N,N'-Bis(neopentoxycarbonyl)-dioxazin der Formel (XIXa) 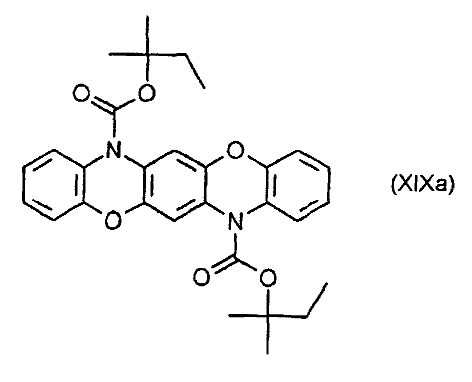 verwendet, welches nach der in EP 648 817 angegebenen Methode hergestellt wurde. Der beschichtete Träger wird dann mit dem fokussierten Strahl eines Ar-lonenlasers (sichtbar, alle Linien; primär 488 und 514 nm) belichtet. Bei einer Laserleistung von 2,5 W und einer Schreibgeschwindigkeit zwischen 75 und 200 mm/s tritt an den beschrifteten Stellen eine Farbänderung zur charakteristischen violetten Farbe des Dioxazin-Pigments der Formel (XIXb):

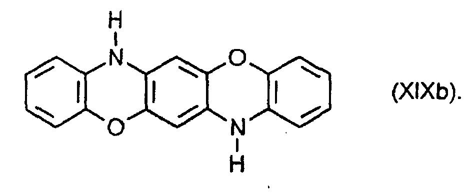

Der Träger wird nachträglich mit 20 ml Diethylether gewaschen, wobei nur die beschrifteten (unlöslichen) Strukturen in Relief auf dem Glasträger bleiben. Die Auflösung ist ≤ 50 µm.

## Patentansprüche

1. Verfahren zur Herstellung eines Materials enthaltend ein Substrat, an dessen Oberfläche mindestens eine aus einem oder mehreren Pigmenten der Formel (I) oder (II) oder Derivaten davon,
**A(D**_{**1**}**)(D**_{**2**}**)**_{**x**} **(I)**
**Pc (II)**
worin A den Rest eines Chromophors der Chinacridon-, Anthrachinon-, Perylen-, Indigo-, Azo-, Chinophthalon-, Isoindolinon-, Isoindolin-, Dioxazin-, Phthalocyanin- oder Diketopyrrolopyrrolreihe bedeutet, der mit D₁ und x D₂ verbundene N-Atome enthält, wobei jedes in A vorhandene N-Atom unabhängig von den anderen mit 0, 1 oder 2 Gruppen D₁ oder D₂ verbunden sein kann,
D₁ und D₂ Wasserstoff, x eine ganze Zahl von 0 bis 4 bedeuten, und
Pc ein Chromophor der Phthalocyanin-Reihe ist,
bestehende Pigmentbeschichtung angebracht ist, durch
(a) Beschichtung des Substrats, vollständig oder nur auf einem Teil desser Oberfläche, mit einer Schmelze mindestens eines latenten Pigmentes der Formel (III) oder eines Derivaten davon,
**A(D**_{**3**}**)(D**_{**4**}**)**_{**x**} **(III)**
oder mindestens eines latenten Pigmentes der Formel (IV) oder eines Stellungsisomeren oder eines Derivaten davon, 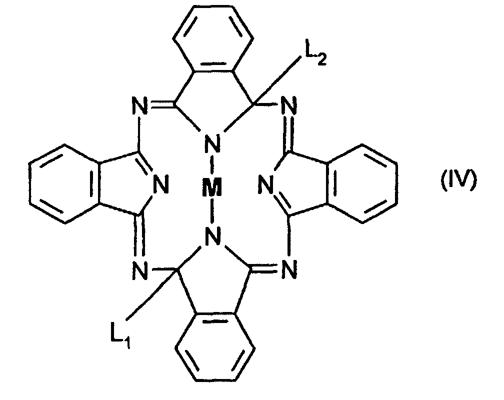 oder mit einer Lösung bestehend aus mindestens einem latenten Pigment der Formel (III), einem Derivaten davon, einem latenten Pigment der Formel (IV), einem Stellungsisomeren oder einem Derivaten davon, und einem oder mehreren Lösungsmitteln,
worin in Formel (III) A und x die gleiche Bedeutung haben wie in Formel (I), wobei A mit D₃ und x D₄ verbundene N-Atome enthält und jedes in A vorhandene N-Atom unabhängig von den anderen mit 0, 1 oder 2 Gruppen D₃ oder D₄ verbunden sein kann, und
D₃ und D₄ unabhängig voneinander Gruppen der Formel 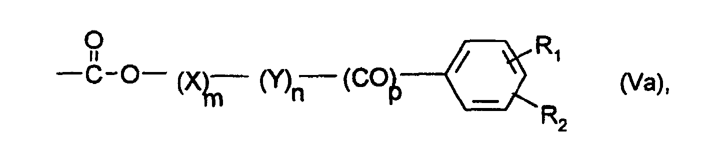 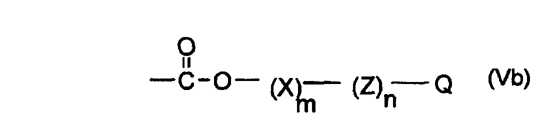 oder 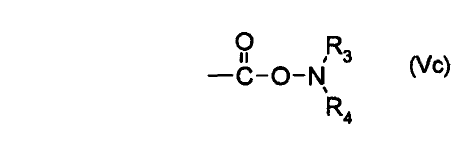 sind,
und worin in Formel (IV) L₁ und L₂ unabhängig voneinander Halogen, C₁-C₁₈-Alkoxy, C₂-C₁₈-Dialkylamino, C₁-C₁₈-Cycloalkylamino, C₁-C₆-Alkylpiperidino oder Morpholino bedeuten, und M für zwei Wasserstoffe oder ein mindestens zweiwertiges Metallatom steht,
und
(b) partielle oder vollständige Überführung des latenten Pigmentes in dessen unlösliche Pigmentform durch Abspaltung der Gruppen D₃ und D₄ unter deren Ersatz durch Wasserstoff, oder durch Abspaltung der Gruppen L₁ und L₂,
wobei in den Formeln (Va), (Vb) und (Vc)
m, n und p unabhängig voneinander 0 oder 1 sind,
X C₁-C₁₄-Alkylen, C₂-C₁₄-Alkenylen, C₂-C₁₄-Alkinylen, C₄-C₁₂-Cycloalkylen oder C₄-C₁₂-Cycloalkenylen,
Y eine Gruppe -V-(CH₂)_{q}-,
Z eine Gruppe -V-(CH₂)ᵣ-,
V C₃-C₆-Cycloalkylen,
q eine Zahl von 1 bis 6 und
r eine Zahl von 0 bis 6 bedeuten,
R₁ und R₂ unabhängig voneinander Wasserstoff, C₁-C₆-Alkyl, C₁-C₄-Alkoxy, Halogen, CN, NO₂, unsubstituiertes oder durch C₁-C₄-Alkyl, C₁-C₄-Alkoxy oder Halogen substituiertes Phenyl oder Phenoxy sind,
Q Wasserstoff, CN, Si(R₁)₃, eine Gruppe C(R₅)(R₆)(R₇),
worin R₅, R₆ und R₇ unabhängig voneinander Wasserstoff oder Halogen sind und mindestens einer der Reste R₅, R₆ und R₇ Halogen bedeutet,
eine Gruppe 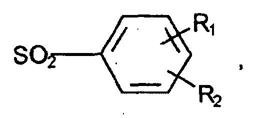 worin R₁ und R₂ die oben angegebene Bedeutung haben,
eine Gruppe SO₂R₈ oder SR₈, worin R₈ C₁-C₄-Alkyl ist,
eine Gruppe CH(R₉)₂, worin R₉ unsubstituiertes oder durch C₁-C₄-Alkyl, C₁-C₄-alkoxy oder Halogen substituiertes Phenyl bedeutet, oder eine Gruppe der Formel 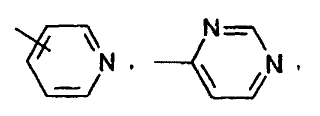 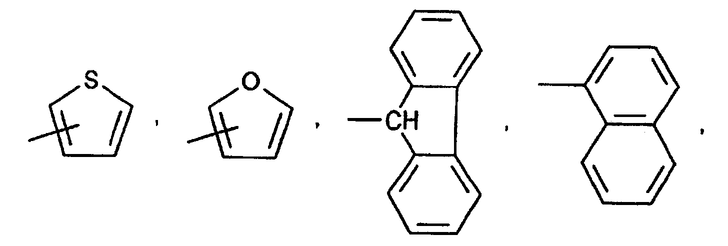 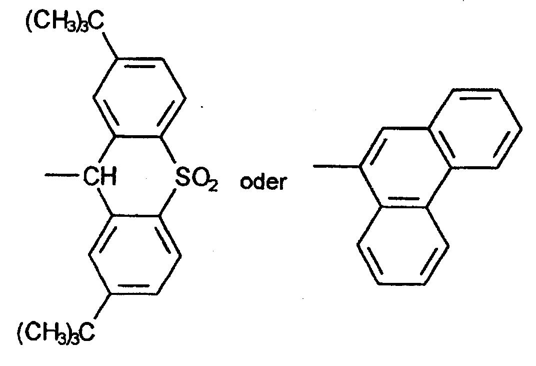 bedeutet, und
R₃ und R₄ unabhängig voneinander Wasserstoff, C₁-C₁₈-Alkyl oder eine Gruppe 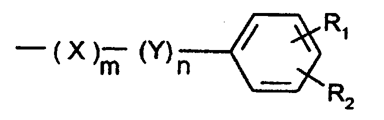 sind,
worin X, Y, R₁, R₂, m und n die oben angegebene Bedeutung haben, oder R₃ und R₄ zusammen mit dem Stickstoffatom an dem sie gebunden sind einen Pyrrolidinyl-, Piperidinyl- oder Morpholinylrest bilden.

2. Verfahren nach Anspruch 1, worin im Anschluss auf Schritte (a) und (b) zusätzlich (c) die Entfernung des übriggebliebenen latenten Pigmentes erfolgt.

3. Verfahren nach Anspruch 1, worin das Substrat in Schritt (a) auf nur einem Teil seiner Oberfläche beschichtet wird.

4. Verfahren nach Anspruch 1, worin Schritt (a) durch Eintauchen, Spritzen, Bedrucken, Giessen, Aufziehen oder Aufschleudern erfolgt.

5. Verfahren nach Anspruch 4, worin Schritt (a) durch Aufschleudern erfolgt.

6. Verfahren nach Anspruch 1, worin Schritt (b) durch mit einem fokussierten Laserstrahl erfolgt.

7. Verfahren nach Anspruch 1, worin Schritt (b) durch Annäherung einer Heizquelle erfolgt.

8. Verfahren nach Anspruch 1, worin Schritt (b) durch Aussetzen dem Dampf einer organischen oder anorganischen Brønsted- oder Lewis-Säure oder einer Base erfolgt.

9. Verfahren nach Anspruch 1, worin Schritt (b) nur auf ausgewählten Gebieten erfolgt.

10. Verfahren nach Anspruch 9, worin Schritt (b) mit einer programmierten Steuerung erfolgt.

11. Verfahren nach Anspruch 2, worin Schritt (c) mit Hilfe eines Lösemittels erfolgt.

12. Verfahren nach Anspruch 2, worin mehrere Pigmentbeschichtungen an der Substratoberfläche angebracht werden, durch aufeinanderfolgendes Anbringen dieser Pigmentbeschichtungen unter jeweiliger Wiederholung der Schritte (a) bis (c) für jede Pigmentbeschichtung.

13. Material enthaltend ein Substrat, an dessen Oberfläche mindestens eine aus einem oder mehreren Pigmenten der Formel (I) oder (II) gemäss Anspruch 1 bestehende Pigmentbeschichtung angebracht ist, **dadurch gekennzeichnet, dass** diese Pigmentbeschichtung an einem Teil der Substratoberfläche im Wesentlichen gleichmässig, und am Rest der Substratoberfläche nicht vorhanden ist.

14. Material nach Anspruch 13, worin mindestens eine Pigmentbeschichtung strukturierte Farbmuster mit einer Auflösung von 10-50 µm aufweist.

15. Material nach Anspruch 13, welches zusätzlich mit einer Schutzschicht versehen ist.

16. Material nach Anspruch 13, worin auf dem Substrat mehrere Pigmentbeschichtungen angebracht sind, welche nicht deckungsgleiche Muster bilden und unterschiedliche Absorptionen und/oder unterschiedliche Absorptionsmaxima aufweisen.

17. Material nach Anspruch 16, worin die Pigmentbeschichtungen aus derartigen Pigmenten bestehen und derart angeordnet sind, dass sich blaue, grüne und rote Gebiete ergeben, und worin das Substrat transparent ist.

18. Material nach Anspruch 13, worin mehrere, gleiche oder gleichfarbige Pigmente enthaltende Pigmentbeschichtungen unterschiedlicher Schichtdicken Muster bilden, so dass die Totalabsorption, die sich durch die Summe der Intensitäten aller sich überlappenden Pigmentbeschichtungen ergibt, an jedem mindestens eine Pigmentbeschichtung aufweisenden Bildpunkt ein Mehrfaches des Wertes der kleinsten Absorption darstellt.

19. Verwendung eines Materials nach Anspruch 13 als Farbfilter.

20. Verwendung eines Materials nach Anspruch 13 zur permanenten Speicherung von digitaler Information.

21. Verfahren zum Ablesen von auf einem Material nach Anspruch 13 gespeicherter digitaler Information durch Bestrahlen mit einer Lichtquelle und Messung der Intensität des reflektierten oder des transmittierten Lichtstrahles.

## Claims

1. A process for the preparation of a material comprising a substrate on whose surface there is at least one pigment coating consisting of one or more pigments of the formula (I) or (II) or derivatives thereof
**A(D**_{**1**}**)(D**_{**2**}**)**_{**x**} **(I)**
**Pc (II)**
in which A is the radical of a chromophore of the quinacridone, anthraquinone, perylene, indigo, azo, quinophthalone, isoindolinone, isoindoline, dioxazine, phthalocyanine or diketopyrrolopyrrole series which contains nitrogen atoms attached to D₁ and to x D₂, each nitrogen atom present in A being able independently of the others to be attached to 0, 1 or 2 groups D₁ or D₂,
D₁ and D₂ are hydrogen, x is an integer from 0 to 4, and
Pc is a chromophore of the phthalocyanine series,
which is applied by
(a) coating the substrate, overall or over only part of its surface, with a melt of at least one latent pigment of the formula (III) or of a derivative thereof,
**A(D**_{**3**}**)(D**_{**4**}**)**_{**x**} **(III)**
or of at least one latent pigment of the formula (IV) or of a positional isomer or of a derivative thereof, 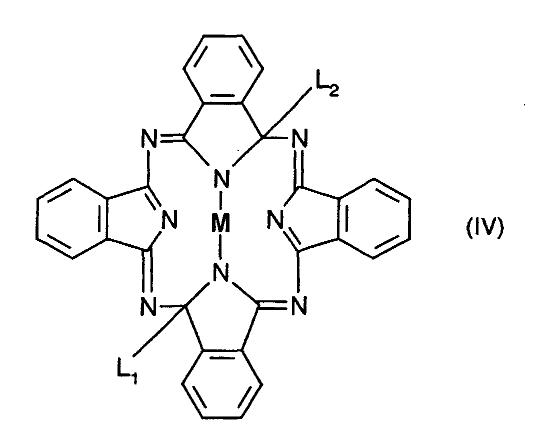 or with a solution consisting of at least one latent pigment of the formula (III), a derivative thereof, a latent pigment of the formula (IV), a positional isomer or a derivative thereof, and one or more solvents,
A and x in formula (III) having the same meaning as in formula (I), A containing nitrogen atoms attached to D₃ and to x D₄, and each nitrogen atom present in A being able independently of the others to be attached to 0, 1 or 2 groups D₃ or D₄, and
D₃ and D₄ independently of one another being groups of the formula 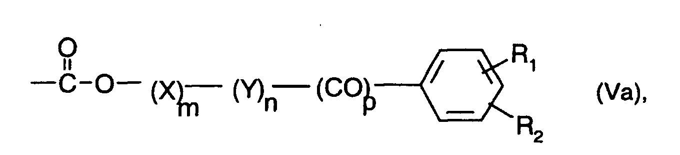 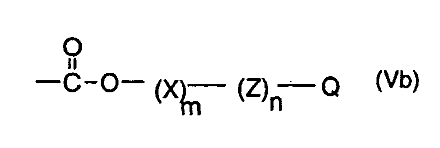 or 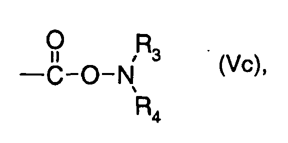 and in formula (IV) L₁ and L₂ independently of one another are halogen, C₁-C₁₈alkoxy, C₂-C₁₈dialkylamino, C₁-C₁₈cycloalkylamino, C₁-C₆alkylpiperidino or morpholino, and M is two hydrogens or an at least divalent metal atom,
and (b) converting the latent pigment partially or completely into its insoluble pigment form by eliminating groups D₃ and D₄ and replacing them by hydrogen, or by eliminating groups L₁ and L₂,
where, in the formulae (Va), (Vb) and (Vc),
m, n and p independently of one another are 0 or 1,
X is C₁-C₁₄alkylene, C₂-C₁₄alkenylene, C₂-C₁₄alkynylene, C₄-C₁₂cycloalkylene or C₄-C₁₂cycloalkenylene,
Y is a group -V-(CH₂)_{q}-,
Z is a group -V-(CH₂)ᵣ-,
V is C₃-C₆cycloalkylene,
q is a number from 1 to 6,
r is a number from 0 to 6,
R₁ and R₂ independently of one another are hydrogen, C₁-C₆alkyl, C₁-C₄alkoxy, halogen, CN, NO₂, phenyl which is unsubstituted or substituted by C₁-C₄alkyl, C₁-C₄alkoxy or halogen, or are phenoxy,
Q is hydrogen, CN, Si(R₁)₃, a group C(R₅)(R₆)(R₇),
in which R₅, R₆ and R₇ independently of one another are hydrogen or halogen and at least one of the radicals R₅, R₆ and R₇ is halogen, a group 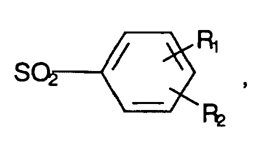 in which R₁ and R₂ are as defined above,
a group SO₂R₈ or SR₈, in which R₈ is C₁-C₄alkyl,
a group CH(R₉)₂, in which R₉ is phenyl which is unsubstituted or substituted by C₁-C₄alkyl, C₁-C₄alkoxy or halogen,
or a group of the formula 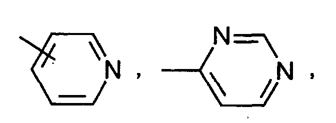 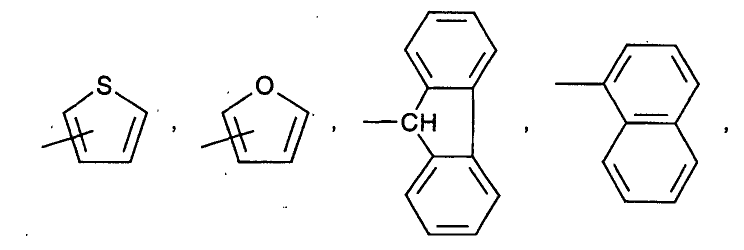 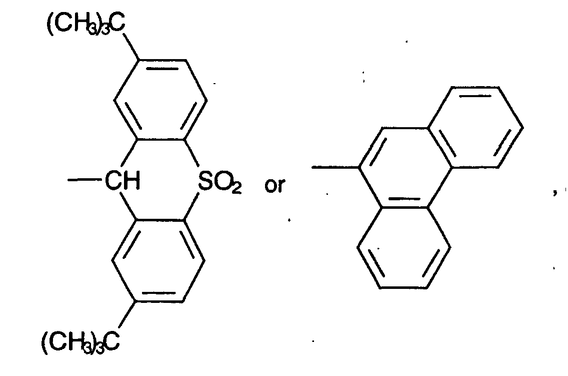 and
R₃ and R₄ independently of one another are hydrogen, C₁-C₁₈alkyl, or a group 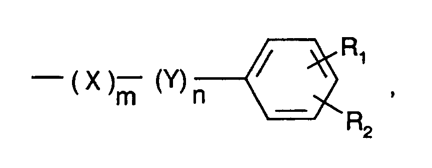 in which X, Y, R₁, R₂, m and n are as defined above, or R₃ and R₄ together with the nitrogen atom to which they are attached form a pyrrolidinyl, piperidyl or morpholinyl radical.

2. A process according to claim 1, wherein steps (a) and (b) are followed by additional step (c) comprising the removal of the remaining latent pigment.

3. A process according to claim 1, wherein the substrate is coated in step (a) over only part of its surface.

4. A process according to claim 1, wherein step (a) is effected by dipping, spraying, printing, curtain coating, knife coating or spin coating.

5. A process according to claim 4, wherein step (a) is effected by spin coating.

6. A process according to claim 1, wherein step (b) is effected by means of a focused laser beam.

7. A process according to claim 1, wherein step (b) is effected by the close action of a heat source.

8. A process according to claim 1, wherein step (b) is effected by exposure to the vapour of an organic or inorganic Bronsted or Lewis acid or of a base.

9. A process according to claim 1, wherein step (b) is effected only at selected areas.

10. A process according to claim 9, wherein step (b) is effected with a programmed control.

11. A process according to claim 2, wherein step (c) is effected with the aid of a solvent.

12. A process according to claim 2, wherein two or more pigment coatings are applied to the substrate surface by successive application of these pigment coatings, repeating steps (a) to (c) for each pigment coating.

13. A material comprising a substrate whose surface carries at least one pigment coating consisting of one or more pigments of the formula (I) or (II) according to claim 1, which coating is substantially uniform on one part of the substrate surface and is absent from the remainder of the substrate surface.

14. A material according to claim 13, wherein at least one pigment coating has structured colour patterns with a resolution of 10-50 µm.

15. A material according to claim 13, which is additionally provided with a protective coat.

16. A material according to claim 13, wherein the substrate is coated with a plurality of pigment coatings which do not form patterns of equal coverage and have different absorptions and/or different absorption maxima.

17. A material according to claim 16, wherein the pigment coatings consist of pigments whose nature and arrangement is such as to give blue, green and red areas and wherein the substrate is transparent.

18. A material according to claim 13, wherein a plurality of pigment coatings of different thicknesses, containing identical or identically coloured pigments, form patterns, so that the total absorption, which is given by the sum of the intensities of all overlapping pigment coatings, at each pixel which has at least one pigment coating is a multiple of the value of the smallest absorption.

19. The use of a material according to claim 13 as a colour filter.

20. The use of a material according to claim 13 for the permanent storage of digital information.

21. A method of reading out digital information stored on a material according to claim 13 by irradiation with a light source and measurement of the intensity of the reflected or transmitted light beam.

## Revendications

1. Procédé de préparation d'un matériau contenant un substrat sur la surface duquel est appliqué au moins un revêtement pigmentaire constitué d'un ou plusieurs pigments de formule (I) ou (II) ou de leurs dérivés,
A(D₁)(D₂)ₓ (I)
Pc (II)
dans lesquelles A représente le reste d'un chromophore de la série des quinacridones, des anthraquinones, des pérylènes, de l'indigo, des colorants azoïques, des quinophtalones, des isoindolinones, des isoindolines, des dioxazines, des phtalocyanines ou des dicétopyrrolopyrroles, contenant des atomes d'azote liés à D₁ et x D₂, chaque atome d'azote présent dans A pouvant être, indépendamment des autres, lié à 0, 1 ou 2 groupes D₁ ou D₂,
D₁ et D₂ représentent des atomes d'hydrogène, x est un nombre entier de 0 à 4, et
Pc est un chromophore de la série des phtalocyanines, par
(a) revêtement du substrat avec une masse fondue d'au moins un pigment latent de formule (III)
A(D₃)(D₄)ₓ (II)
ou un de ses dérivés,
ou d'au moins un pigment latent de formule (IV) 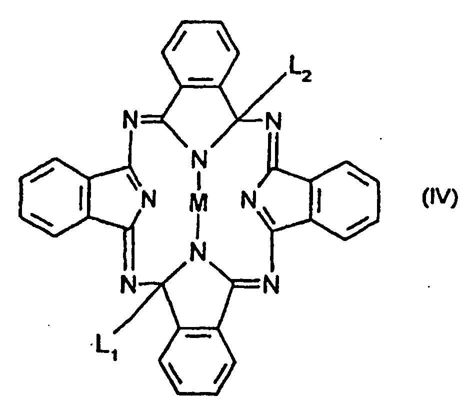 ou un de ses isomères de position ou un de ses dérivés,
ou avec une solution constituée d'au moins un pigment latent de formule (III), un de ses dérivés, un pigment latent de formule (IV), un de ses isomères de position ou un de ses dérivés, et d'un ou plusieurs solvants,
où, dans la formule (III), A et x ont la même signification que dans la formule (I), A contenant des atomes d'azote liés à D₃ et x D₄, et chaque atome d'azote présent dans A pouvant être, indépendamment des autres, lié à 0, 1 ou 2 groupes D₃ ou D₄, et
D₃ et D₄ représentent, indépendamment l'un de l'autre, des groupes de formule 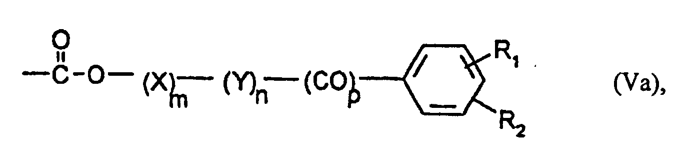 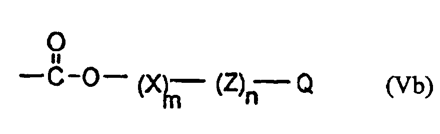 ou 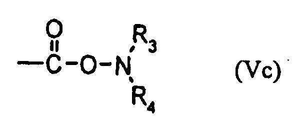 et où, dans la formule (IV), L₁ et L₂ représentent indépendamment l'un de l'autre un un groupe halogéno ou alcoxy en C₁-C₁₈, dialkylamino en C₂-C₁₈, cycloalkylamino en C₁-C₁₈, (alkyl en C₁-C₆)pipéridino ou morpholino, et M représente deux atomes d'hydrogène ou un atome de métal au moins divalent, et
(b) transformation partielle ou totale du pigment latent en sa forme de pigment insoluble par séparation des groupes D₃ et D₄ et leur remplacement par de l'hydrogène, ou par séparation des groupes L₁ et L₂,
et où, dans les formules (Va), (Vb) et (Vc)
m, n et p sont indépendamment les uns des autres égaux à 0 ou 1,
X représente un groupe alkylène en C₁-C₁₄, alcénylène en C₂-C₁₄, alcynylène en C₂-C₁₄, cycloalkylène en C₄-C₁₂ ou cycloalcénylène en C₄-C₁₂,
Y représente un groupe -V-(CH₂)_{q}-,
Z représente un groupe -V-(CH₂)ᵣ-,
V représente un groupe cycloalkylène en C₃-C₆,
q représente un nombre de 1 à 6 et
r représente un nombre de 0 à 6,
R₁ et R₂ représentent, indépendamment l'un de l'autre, un atome d'hydrogène, un groupe alkyle en C₁-C₆, alcoxy en C₁-C₄, halogéno, CN ou NO₂ ou un groupe phényle ou phénoxy non substitué ou substitué par alkyle en C₁-C₄, alcoxy en C₁-C₄ ou halogéno,
Q représente un atome d'hydrogène, un groupe CN, un groupe Si(R₁)₃, un groupe C(R₅)(R₆)(R₇), dans lequel R₅, R₆ et R₇ représentent indépendamment les uns des autres un atome d'hydrogène ou d'halogène et au moins l'un des restes R₅, R₆ et R₇ est un halogène,
un groupe 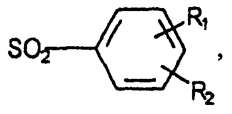 dans lequel R₁ et R₂ ont la signification indiquée ci-dessus,
un groupe SO₂R₈ ou SR₈ dans lequel R₈ est un groupe alkyle en C₁-C₄,
un groupe CH(R₉)₂, dans lequel R₉ représente un groupe phényle non substitué ou substitué par alkyle en C₁-C₄, alcoxy en C₁-C₄ ou halogéno,
ou un groupe de formule 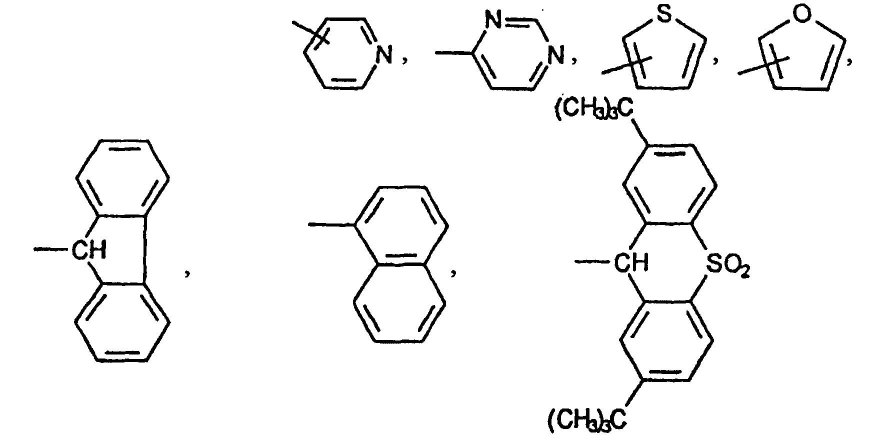 ou 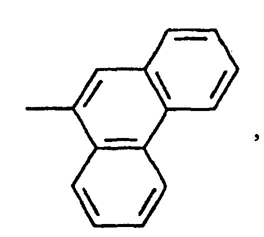 et
R₃ et R₄ représentent, indépendamment l'un de l'autre, un atome d'hydrogène, un groupe alkyle en C₁-C₁₈ ou un groupe 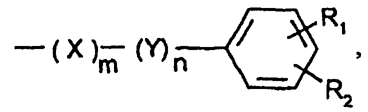 dans lequel X, Y, R₁, R₂, m et n ont la signification indiquée ci-dessus, ou R₃ et R₄ forment ensemble, avec l'atome d'azote auquel ils sont liés, un reste pyrrolidinyle, pipéridinyle ou morpholinyle.

2. Procédé selon la revendication 1, dans lequel, après les étapes (a) et (b) on procède en outre à (c) l'élimination du pigment latent résiduel.

3. Procédé selon la revendication 1, dans lequel le substrat n'est revêtu dans l'étape (a) que sur une partie de sa surface.

4. Procédé selon la revendication 1, dans lequel l'étape (a) s'effectue par immersion, pulvérisation, impression, coulée, absorption ou centrifugation.

5. Procédé selon la revendication 4, dans lequel l'étape (a) se fait par centrifugation.

6. Procédé selon la revendication 1, dans lequel l'étape (b) s'effectue à l'aide d'un rayon laser focalisé.

7. Procédé selon la revendication 1, dans lequel on effectue l'étape (b) en approchant une source de chaleur.

8. Procédé selon la revendication 1, dans lequel l'étape (b) s'effectue par exposition à la vapeur d'un acide de Brønsted ou de Lewis organique ou inorganique ou d'une base.

9. Procédé selon la revendication 1, dans lequel l'étape (b) ne s'effectue que sur des zones choisies.

10. Procédé selon la revendication 9, dans lequel l'étape (b) s'effectue avec une commande programmée.

11. Procédé selon la revendication 2, dans lequel l'étape (c) s'effectue à l'aide d'un solvant.

12. Procédé selon la revendication 2, dans lequel plusieurs revêtements pigmentaires sont appliqués sur la surface du substrat par application successive de ces revêtements pigmentaires avec à chaque fois la répétition des étapes (a) à (c) pour chaque revêtement pigmentaire.

13. Matériau contenant un substrat sur la surface duquel est appliqué au moins un revêtement pigmentaire constitué d'un ou plusieurs pigments de formule (I) ou (II) selon la revendication 1, **caractérisé en ce que** ce revêtement pigmentaire est essentiellement uniforme sur une portion de la surface du substrat, et n'est pas présent sur le reste de la surface du substrat.

14. Matériau selon la revendication 13, dans lequel au moins un revêtement pigmentaire présente un dessin coloré structuré avec une définition de 10 à 50 µm.

15. Matériau selon la revendication 13, qui est muni en outre d'une couche protectrice.

16. Matériau selon la revendication 13, dans lequel plusieurs revêtements pigmentaires ne formant pas le même dessins et ayant des absorptions différentes et/ou des maximums d'absorption différents sont appliqués sur le substrat.

17. Matériau selon la revendication 16, dans lequel les revêtements pigmentaires sont constitués de pigments tels et sont disposés d'une façon telle qu'il se forme des domaines bleus, verts et rouges, et le substrat est transparent.

18. Matériau selon la revendication 13, dans lequel plusieurs revêtements pigmentaires d'épaisseur de couche différente, contenant des pigments identiques ou de même couleur, forment des dessins de façon que l'absorption totale, obtenue par l'addition des intensités de tous les revêtements pigmentaires qui se recouvrent, représente au niveau de chaque élément d'image présentant au moins un revêtement pigmentaire un multiple de la valeur de la plus petite absorption.

19. Utilisation d'un matériau selon la revendication 13 comme filtre couleur.

20. Utilisation d'un matériau selon la revendication 13 pour l'enregistrement permanent d'informations numériques.

21. Procédé de lecture d'informations numériques enregistrées sur un matériau selon la revendication 13 par exposition à une source de lumière et mesure de l'intensité du rayon lumineux réfléchi ou transmis.
